# EUROPEAN PATENT SPECIFICATION

(11) **EP 3 616 727 B1**
(45) Date of publication and mention of the grant of the patent: **31.03.2021**
(21) Application number: 19197388.2
(22) Date of filing: 26.02.2014
(51) Int. Cl.: A61K 47/68, C07K 14/62

(54) **INSULIN ANALOG CONJUGATES AND USE THEREOF**
INSULINANALOGKONJUGATE UND DEREN VERWENDUNG
CONJUGUÉS D'ANALOGUE DE L'INSULINE ET LEURS UTILISATIONS

(30) Priority: 26.02.2013 KR 20130020703; 12.07.2013 KR 20130082511; 20.01.2014 KR 20140006937
(43) Date of publication of application: 04.03.2020
(62) Divisional of application: 14757629.2
(73) Proprietor: Hanmi Pharm. Co., Ltd., Hwaseong-si, Gyeonggi-do 445-958 (KR)
(72) Inventor: HWANG, Sang Youn, 445-855 Gyeonggi-do (KR); HUH, Yong Ho, 156-744 Seoul (KR); KIM, Jin Young, 134-781 Seoul (KR); HONG, Sung Hee, 443-769 Gyeonggi-do (KR); CHOI, In Young, 448-160 Gyeonggi-do (KR); JUNG, Sung Youb, 442-754 Gyeonggi-do (KR); KWON, Se Chang, 135-506 Seoul (KR); KIM, Dae Jin, 445-764 Gyeonggi-do (KR); KIM, Hyun Uk, 600-810 Busan (KR); JANG, Myung Hyun, 134-715 Seoul (KR); KIM, Seung Su, 18469 Gyeonggi-do (KR)
(74) Representative: Mewburn Ellis LLP

(56) References cited:
- WO-A2-2011/122921
- KR-A- 20110 134 209
- KRISTENSEN C ET AL: "Alanine Scanning Mutagenesis of Insulin", JOURNAL OF BIOLOGICAL CHEMISTRY, AMERICAN SOCIETY FOR BIOCHEMISTRY AND MOLECULAR BIOLOGY, US, vol. 272, no. 20, 16 May 1997 (1997-05-16) , pages 12978-12983, XP002106057, ISSN: 0021-9258, DOI: 10.1074/JBC.272.20.12978
- HINDS K D ET AL: "Effects of PEG conjugation of insulin properties", ADVANCED DRUG DELIVERY REVIEWS, ELSEVIER, AMSTERDAM, NL, vol. 54, 1 January 2002 (2002-01-01), pages 505-530, XP003004966, ISSN: 0169-409X, DOI: 10.1016/S0169-409X(02)00025-X
- THIBAUDEAU KAREN ET AL: "Synthesis and evaluation of insulin-human serum albumin conjugates", BIOCONJUGATE CHEMISTRY, AMERICAN CHEMICAL SOCIETY, US, vol. 16, no. 4, 25 June 2005 (2005-06-25), pages 1000-1008, XP002405343, ISSN: 1043-1802
- BAUDYS M ET AL: "Extending insulin action in vivo by conjugation to carboxymethyl dextran", BIOCONJUGATE CHEMISTRY, AMERICAN CHEMICAL SOCIETY, US, vol. 9, no. 2, 5 February 1998 (1998-02-05), pages 176-183, XP002363506, ISSN: 1043-1802, DOI: 10.1021/BC970180A

<110> HANMI PHARM. CO., LTD.
<120> Novel insulin analog and use thereof
<130> OPA14031-PCT
<150> KR 10-2013-0020703
   <151> 2013-02-26
<150> KR 10-2013-0082511
   <151> 2013-07-12
<150> KR 10-2014-0006937
   <151> 2014-01-20
<160> 38
<170> KopatentIn 2.0
<210> 1
   <211> 39
   <212> DNA
   <213> Artificial Sequence
<220>
   <223> Primer
<400> 1
   gggtccctgc agaagcgtgc gattgtggaa caatgctgt 39
<210> 2
   <211> 39
   <212> DNA
   <213> Artificial Sequence
<220>
   <223> Primer
<400> 2
   acagcattgt tccacaatcg cacgcttctg cagggaccc 39
<210> 3
   <211> 39
   <212> DNA
   <213> Artificial Sequence
<220>
   <223> Primer
<400> 3
   tccctgcaga agcgtggcgc ggtggaacaa tgctgtacc 39
<210> 4
   <211> 39
   <212> DNA
   <213> Artificial Sequence
<220>
   <223> Primer
<400> 4
   ggtacagcat tgttccaccg cgccacgctt ctgcaggga 39
<210> 5
   <211> 36
   <212> DNA
   <213> Artificial Sequence
<220>
   <223> Primer
<400> 5
   ctctaccagc tggaaaacgc gtgtaactga ggatcc 36
<210> 6
   <211> 36
   <212> DNA
   <213> Artificial Sequence
<220>
   <223> Primer
<400> 6
   ggatcctcag ttacacgcgt tttccagctg gtagag 36
<210> 7
   <211> 39
   <212> DNA
   <213> Artificial Sequence
<220>
   <223> Primer
<400> 7
   gttaaccaac acttgtgtgc gtcacacctg gtggaagct 39
<210> 8
   <211> 39
   <212> DNA
   <213> Artificial Sequence
<220>
   <223> Primer
<400> 8
   agcttccacc aggtgtgacg cacacaagtg ttggttaac 39
<210> 9
   <211> 39
   <212> DNA
   <213> Artificial Sequence
<220>
   <223> Primer
<400> 9
   ctagtgtgcg gggaacgagc gttcttctac acacccaag 39
<210> 10
   <211> 39
   <212> DNA
   <213> Artificial Sequence
<220>
   <223> Primer
<400> 10
   cttgggtgtg tagaagaacg ctcgttcccc gcacactag 39
<210> 11
   <211> 39
   <212> DNA
   <213> Artificial Sequence
<220>
   <223> Primer
<400> 11
   gtgtgcgggg aacgaggcgc gttctacaca cccaagacc 39
<210> 12
   <211> 39
   <212> DNA
   <213> Artificial Sequence
<220>
   <223> Primer
<400> 12
   ggtcttgggt gtgtagaacg cgcctcgttc cccgcacac 39
<210> 13
   <211> 39
   <212> DNA
   <213> Artificial Sequence
<220>
   <223> Primer
<400> 13
   tgcggggaac gaggcttcgc gtacacaccc aagacccgc 39
<210> 14
   <211> 39
   <212> DNA
   <213> Artificial Sequence
<220>
   <223> Primer
<400> 14
   gcgggtcttg ggtgtgtacg cgaagcctcg ttccccgca 39
<210> 15
   <211> 37
   <212> DNA
   <213> Artificial Sequence
<220>
   <223> Primer
<400> 15
   ccagcatctg ctccctcgaa cagctggaga actactg 37
<210> 16
   <211> 37
   <212> DNA
   <213> Artificial Sequence
<220>
   <223> Primer
<400> 16
   cagtagttct ccagctgttc gagggagcag atgctgg 37
<210> 17
   <211> 34
   <212> DNA
   <213> Artificial Sequence
<220>
   <223> Primer
<400> 17
   cagcatctgc tccctcaacc agctggagaa ctac 34
<210> 18
   <211> 34
   <212> DNA
   <213> Artificial Sequence
<220>
   <223> Primer
<400> 18
   gtagttctcc agctggttga gggagcagat gctg 34
<210> 19
   <211> 258
   <212> DNA
   <213> Artificial Sequence
<220>
   <223> Analog 1
<400> 19
   ttcgttaacc aacacttgtg tggctcacac ctggtggaag ctctctacct agtgtgcggg    60
   gaacgaggct tcttctacac acccaagacc cgccgggagg cagaggacct gcaggtgggg   120
   caggtggagc tgggcggggg ccctggtgca ggcagcctgc agcccttggc cctggagggg   180
   tccctgcaga agcgtgcgat tgtggaacaa tgctgtacca gcatctgctc cctctaccag   240
   ctggagaact actgcaac                                                 258
<210> 20
   <211> 86
   <212> PRT
   <213> Artificial Sequence
<220>
   <223> Analog 1
<400> 20
   Phe Val Asn Gln His Leu Cys Gly Ser His Leu Val Glu Ala Leu Tyr
   1               5                   10                  15
   Leu Val Cys Gly Glu Arg Gly Phe Phe Tyr Thr Pro Lys Thr Arg Arg
               20                  25                  30
   Glu Ala Glu Asp Leu Gln Val Gly Gln Val Glu Leu Gly Gly Gly Pro
           35                  40                  45
   Gly Ala Gly Ser Leu Gln Pro Leu Ala Leu Glu Gly Ser Leu Gln Lys
       50                  55                  60
   Arg Ala Ile Val Glu Gln Cys Cys Thr Ser Ile Cys Ser Leu Tyr Gln
   65                  70                  75                  80
   Leu Glu Asn Tyr Cys Asn
                   85
<210> 21
   <211> 258
   <212> DNA
   <213> Artificial Sequence
<220>
   <223> Analog 2
<400> 21
   ttcgttaacc aacacttgtg tggctcacac ctggtggaag ctctctacct agtgtgcggg     60
   gaacgaggct tcttctacac acccaagacc cgccgggagg cagaggacct gcaggtgggg    120
   caggtggagc tgggcggggg ccctggtgca ggcagcctgc agcccttggc cctggagggg    180
   tccctgcaga agcgtggcgc ggtggaacaa tgctgtacca gcatctgctc cctctaccag    240
   ctggagaact actgcaac                                                  258
<210> 22
   <211> 86
   <212> PRT
   <213> Artificial Sequence
<220>
   <223> Analog 2
<400> 22
   Phe Val Asn Gln His Leu Cys Gly Ser His Leu Val Glu Ala Leu Tyr
    1               5                  10                  15
   Leu Val Cys Gly Glu Arg Gly Phe Phe Tyr Thr Pro Lys Thr Arg Arg
               20                  25                  30
   Glu Ala Glu Asp Leu Gln Val Gly Gln Val Glu Leu Gly Gly Gly Pro
           35                  40                  45
   Gly Ala Gly Ser Leu Gln Pro Leu Ala Leu Glu Gly Ser Leu Gln Lys
       50                  55                  60
   Arg Gly Ala Val Glu Gln Cys Cys Thr Ser Ile Cys Ser Leu Tyr Gln
   65                  70                  75                  80
   Leu Glu Asn Tyr Cys Asn
                   85
<210> 23
   <211> 258
   <212> DNA
   <213> Artificial Sequence
<220>
   <223> Analog 3
<400> 23
   ttcgttaacc aacacttgtg tggctcacac ctggtggaag ctctctacct agtgtgcggg      60
   gaacgaggct tcttctacac acccaagacc cgccgggagg cagaggacct gcaggtgggg     120
   caggtggagc tgggcggggg ccctggtgca ggcagcctgc agcccttggc cctggagggg     180
   tccctgcaga agcgtggcat tgtggaacaa tgctgtacca gcatctgctc cctctaccag     240
   ctggagaacg cgtgcaac                                                   258
<210> 24
   <211> 86
   <212> PRT
   <213> Artificial Sequence
<220>
   <223> Analog 3
<400> 24
   Phe Val Asn Gln His Leu Cys Gly Ser His Leu Val Glu Ala Leu Tyr
   1 5 10 15
   Leu Val Cys Gly Glu Arg Gly Phe Phe Tyr Thr Pro Lys Thr Arg Arg
   20 25 30
   Glu Ala Glu Asp Leu Gln Val Gly Gln Val Glu Leu Gly Gly Gly Pro
   35 40 45
   Gly Ala Gly Ser Leu Gln Pro Leu Ala Leu Glu Gly Ser Leu Gln Lys
   50 55 60
   Arg Gly Ile Val Glu Gln Cys Cys Thr Ser Ile Cys Ser Leu Tyr Gln
   65 70 75 80
   Leu Glu Asn Ala Cys Asn
   85
<210> 25
   <211> 258
   <212> DNA
   <213> Artificial Sequence
<220>
   <223> Analog 4
<400> 25
   ttcgttaacc aacacttgtg tgcgtcacac ctggtggaag ctctctacct agtgtgcggg 60
   gaacgaggct tcttctacac acccaagacc cgccgggagg cagaggacct gcaggtgggg 120
   caggtggagc tgggcggggg ccctggtgca ggcagcctgc agcccttggc cctggagggg 180
   tccctgcaga agcgtggcat tgtggaacaa tgctgtacca gcatctgctc cctctaccag 240
   ctggagaact actgcaac 258
<210> 26
   <211> 86
   <212> PRT
   <213> Artificial Sequence
<220>
   <223> Analog 4
<400> 26
   Phe Val Asn Gln His Leu Cys Ala Ser His Leu Val Glu Ala Leu Tyr
   1 5 10 15
   Leu Val Cys Gly Glu Arg Gly Phe Phe Tyr Thr Pro Lys Thr Arg Arg
   20 25 30
   Glu Ala Glu Asp Leu Gln Val Gly Gln Val Glu Leu Gly Gly Gly Pro
               35                  40                  45
   Gly Ala Gly Ser Leu Gln Pro Leu Ala Leu Glu Gly Ser Leu Gln Lys
       50                  55                  60
   Arg Gly Ile Val Glu Gln Cys Cys Thr Ser Ile Cys Ser Leu Tyr Gln
   65                  70                  75                  80
   Leu Glu Asn Tyr Cys Asn
                   85
<210> 27
   <211> 258
   <212> DNA
   <213> Artificial Sequence
<220>
   <223> Analog 5
<400> 27
   ttcgttaacc aacacttgtg tggctcacac ctggtggaag ctctctacct agtgtgcggg      60
   gaacgagcgt tcttctacac acccaagacc cgccgggagg cagaggacct gcaggtgggg     120
   caggtggagc tgggcggggg ccctggtgca ggcagcctgc agcccttggc cctggagggg     180
   tccctgcaga agcgtggcat tgtggaacaa tgctgtacca gcatctgctc cctctaccag     240
   ctggagaact actgcaac                                                   258
<210> 28
   <211> 86
   <212> PRT
   <213> Artificial Sequence
<220>
   <223> Analog 5
<400> 28
   Phe Val Asn Gln His Leu Cys Gly Ser His Leu Val Glu Ala Leu Tyr
   1               5                   10                  15
   Leu Val Cys Gly Glu Arg Ala Phe Phe Tyr Thr Pro Lys Thr Arg Arg
               20                  25                  30
   Glu Ala Glu Asp Leu Gln Val Gly Gln Val Glu Leu Gly Gly Gly Pro
           35                  40                  45
   Gly Ala Gly Ser Leu Gln Pro Leu Ala Leu Glu Gly Ser Leu Gln Lys
       50                  55                  60
   Arg Gly Ile Val Glu Gln Cys Cys Thr Ser Ile Cys Ser Leu Tyr Gln
   65                  70                  75                  80
   Leu Glu Asn Tyr Cys Asn
                   85
<210> 29
   <211> 258
   <212> DNA
   <213> Artificial Sequence
<220>
   <223> Analog 6
<400> 29
   ttcgttaacc aacacttgtg tggctcacac ctggtggaag ctctctacct agtgtgcggg      60
   gaacgaggcg cgttctacac acccaagacc cgccgggagg cagaggacct gcaggtgggg     120
   caggtggagc tgggcggggg ccctggtgca ggcagcctgc agcccttggc cctggagggg     180
   tccctgcaga agcgtggcat tgtggaacaa tgctgtacca gcatctgctc cctctaccag     240
   ctggagaact actgcaac                                                   258
<210> 30
   <211> 86
   <212> PRT
   <213> Artificial Sequence
<220>
   <223> Analog 6
<400> 30
   Phe Val Asn Gln His Leu Cys Gly Ser His Leu Val Glu Ala Leu Tyr
   1               5                   10                  15
   Leu Val Cys Gly Glu Arg Gly Ala Phe Tyr Thr Pro Lys Thr Arg Arg
               20                  25                  30
   Glu Ala Glu Asp Leu Gln Val Gly Gln Val Glu Leu Gly Gly Gly Pro
           35                  40                  45
   Gly Ala Gly Ser Leu Gln Pro Leu Ala Leu Glu Gly Ser Leu Gln Lys
       50                  55                  60
   Arg Gly Ile Val Glu Gln Cys Cys Thr Ser Ile Cys Ser Leu Tyr Gln
   65                  70                  75                  80
   Leu Glu Asn Tyr Cys Asn
                   85
<210> 31
   <211> 258
   <212> DNA
   <213> Artificial Sequence
<220>
   <223> Analog 7
<400> 31
   ttcgttaacc aacacttgtg tggctcacac ctggtggaag ctctctacct agtgtgcggg 60
   gaacgaggct tcgcgtacac acccaagacc cgccgggagg cagaggacct gcaggtgggg 120
   caggtggagc tgggcggggg ccctggtgca ggcagcctgc agcccttggc cctggagggg 180
   tccctgcaga agcgtggcat tgtggaacaa tgctgtacca gcatctgctc cctctaccag 240
   ctggagaact actgcaac 258
<210> 32
   <211> 86
   <212> PRT
   <213> Artificial Sequence
<220>
   <223> Analog 7
<400> 32
   Phe Val Asn Gln His Leu Cys Gly Ser His Leu Val Glu Ala Leu Tyr
   1 5 10 15
   Leu Val Cys Gly Glu Arg Gly Phe Ala Tyr Thr Pro Lys Thr Arg Arg
   20 25 30
   Glu Ala Glu Asp Leu Gln Val Gly Gln Val Glu Leu Gly Gly Gly Pro
   35 40 45
   Gly Ala Gly Ser Leu Gln Pro Leu Ala Leu Glu Gly Ser Leu Gln Lys
   50 55 60
   Arg Gly Ile Val Glu Gln Cys Cys Thr Ser Ile Cys Ser Leu Tyr Gln
   65 70 75 80
   Leu Glu Asn Tyr Cys Asn
   85
<210> 33
   <211> 261
   <212> DNA
   <213> Artificial Sequence
<220>
   <223> Analog 8
<400> 33
   ttcgttaacc aacacttgtg tggctcacac ctggtggaag ctctctacct agtgtgcggg 60
   gaacgaggct tcttctacac acccaagacc cgccgggagg cagaggacct gcaggtgggg 120
   caggtggagc tgggcggggg ccctggtgca ggcagcctgc agcccttggc cctggagggg 180
   tccctgcaga agcgtggcat tgtggaacaa tgctgtacca gcatctgctc cctcgaacag 240
   ctggagaact actgcaactg a 261
<210> 34
   <211> 86
   <212> PRT
   <213> Artificial Sequence
<220>
   <223> Analog 8
<400> 34
   Phe Val Asn Gln His Leu Cys Gly Ser His Leu Val Glu Ala Leu Tyr
   1               5                   10                  15
   Leu Val Cys Gly Glu Arg Gly Phe Phe Tyr Thr Pro Lys Thr Arg Arg
               20                  25                  30
   Glu Ala Glu Asp Leu Gln Val Gly Gln Val Glu Leu Gly Gly Gly Pro
           35                  40                  45
   Gly Ala Gly Ser Leu Gln Pro Leu Ala Leu Glu Gly Ser Leu Gln Lys
       50                  55                  60
   Arg Gly Ile Val Glu Gln Cys Cys Thr Ser Ile Cys Ser Leu Glu Gln
   65                  70                  75                  80
   Leu Glu Asn Tyr Cys Asn
                   85
<210> 35
   <211> 261
   <212> DNA
   <213> Artificial Sequence
<220>
   <223> Analog 9
<400> 35
   ttcgttaacc aacacttgtg tggctcacac ctggtggaag ctctctacct agtgtgcggg      60
   gaacgaggct tcttctacac acccaagacc cgccgggagg cagaggacct gcaggtgggg     120
   caggtggagc tgggcggggg ccctggtgca ggcagcctgc agcccttggc cctggagggg     180
   tccctgcaga agcgtggcat tgtggaacaa tgctgtacca gcatctgctc cctcaaccag     240
   ctggagaact actgcaactg a                                               261
<210> 36
   <211> 86
   <212> PRT
   <213> Artificial Sequence
<220>
   <223> Analog 9
<400> 36
   Phe Val Asn Gln His Leu Cys Gly Ser His Leu Val Glu Ala Leu Tyr
    1               5                  10                  15
   Leu Val Cys Gly Glu Arg Gly Phe Phe Tyr Thr Pro Lys Thr Arg Arg
               20                  25                  30
   Glu Ala Glu Asp Leu Gln Val Gly Gln Val Glu Leu Gly Gly Gly Pro
           35                  40                  45
   Gly Ala Gly Ser Leu Gln Pro Leu Ala Leu Glu Gly Ser Leu Gln Lys
       50                  55                  60
   Arg Gly Ile Val Glu Gln Cys Cys Thr Ser Ile Cys Ser Leu Asn Gln
   65                  70                  75                  80
   Leu Glu Asn Tyr Cys Asn
                   85
<210> 37
   <211> 21
   <212> PRT
   <213> Artificial Sequence
<220>
   <223> A chain of insulin
<400> 37
   Gly Ile Val Glu Gln Cys Cys Thr Ser Ile Cys Ser Leu Tyr Gln Leu
    1               5                  10                  15
   Glu Asn Tyr Cys Asn
               20
<210> 38
   <211> 30
   <212> PRT
   <213> Artificial Sequence
<220>
   <223> B chain of insulin
<400> 38
   Phe Val Asn Gln His Leu Cys Gly Ser His Leu Val Glu Ala Leu Tyr
    1               5                  10                  15
   Leu Val Cys Gly Glu Arg Gly Phe Phe Tyr Thr Pro Lys Thr
               20                  25                  30

## Description

### [Technical Field]

The present disclosure relates to an insulin analog that has a reduced insulin titer and a reduced insulin receptor binding affinity compared to the native form for the purpose of increasing the blood half-life of insulin, a conjugate prepared by linking the insulin analog and a carrier, a long-acting formulation including the conjugate, and a method for preparing the conjugate.

### [Background Art]

In vivo proteins are known to be eliminated via various routes, such as degradation by proteolytic enzymes in blood, excretion through the kidney, or clearance by receptors. Thus, many efforts have been made to improve therapeutic efficacy by avoiding the protein clearance mechanisms and increasing half-life of physiologically active proteins.

On the other hand, insulin is a hormone secreted by the pancreas of the human body, which regulates blood glucose levels, and plays a role in maintaining normal blood glucose levels while carrying surplus glucose in the blood to cells to provide energy for cells. In diabetic patients, however, insulin does not function properly due to lack of insulin, resistance to insulin, and loss of beta-cell function, and thus glucose in the blood cannot be utilized as an energy source and the blood glucose level is elevated, leading to hyperglycemia. Eventually, urinary excretion occurs, contributing to development of various complications. Therefore, insulin therapy is essential for patients with abnormal insulin secretion (Type I) or insulin resistance (Type II), and blood glucose levels can be normally regulated by insulin administration. However, like other protein and peptide hormones, insulin has a very short in vivo half-life, and thus has a disadvantage of repeated administration. Such frequent administration causes severe pain and discomfort for the patients. For this reason, in order to improve quality of life by increasing in vivo half-life of the protein and reducing the administration frequency, many studies on protein formulation and chemical conjugation (fatty acid conjugate, polyethylene polymer conjugate) have been conducted. Commercially available long-acting insulin includes insulin glargine manufactured by Sanofi Aventis (lantus, lasting for about 20-22 hours), and insulin detemir (levemir, lasting for about 18-22 hours) and tresiba (degludec, lasting for about 40 hours) manufactured by Novo Nordisk. These long-acting insulin formulations produce no peak in the blood insulin concentration, and thus they are suitable as basal insulin. However, because these formulations do not have sufficiently long half-life, the disadvantage of one or two injections per day still remains. Accordingly, there is a limitation in achieving the intended goal that administration frequency is remarkably reduced to improve convenience of diabetic patients in need of long-term administration.

The previous research reported a specific in vivo insulin clearance process; 50% or more of insulin is removed in the kidney and the rest is removed via a receptor mediated clearance (RMC) process in target sites such as muscle, fat, liver, etc.

In this regard, many studies, including J Pharmacol Exp Ther (1998) 286: 959, Diabetes Care (1990) 13: 923, Diabetes (1990) 39: 1033, have reported that in vitro activity is reduced to avoid RMC of insulin, thereby increasing the blood level. However, these insulin analogs having reduced receptor binding affinity cannot avoid renal clearance which is a main clearance mechanism, although RMC is reduced. Accordingly, there has been a limit in remarkably increasing the blood half-life.

Kristensen et al., J. Biol. Chem., 272 (20), 12978 - 12983 (1997) relates to "Alanine scanning mutagenesis of insulin", and in particular the finding that "B20Ala insulin retains high affinity binding to the receptor."

WO 2011/122921 relates to "an insulin conjugate having improved in vivo duration and stability, which is prepared by covalently linking insulin with an immunoglobulin Fc region via a non-peptidyl polymer."

KR10-2011-0134209 relates to "A single chain-insulin analog complex using an immunoglobulin fragment."

Hinds & Kim, Adv. Drug Delivery Reviews, 54, 505-530 (2002) relates to the "Effects of PEG conjugation on insulin properties".

Thibaudeau et al., Bioconjugate Chem., 16 (4), 1000-1008 (2005) relates to "synthesis and evaluation of insulin-human serum albumin conjugates".

Baudys et al., Bioconjugate Chem., 9 (2), 176-183 (1998) is an investigation into means of "extending insulin action in vivo by conjugation to carboxymethyl dextran".

Under this background, the present authors have made many efforts to increase the blood half-life of insulin. As a result, they found that a novel insulin analog having no native insulin sequence but a non-native insulin sequence shows a reduced in-vitro titer and a reduced insulin receptor binding affinity, and therefore, its renal clearance can be reduced. They also found that the blood half-life of insulin can be further increased by linking the insulin analog to an immunoglobulin Fc fragment as a representative carrier effective for half-life improvement.

### [Disclosure]

### [Technical Problem]

An object of the present disclosure is to provide an insulin analog that is prepared to have a reduced in-vitro titer for the purpose of prolonging in vivo half-life of insulin, and a conjugate prepared by linking a carrier thereto.

Specifically, one object of the present disclosure is to provide an insulin analog having a reduced insulin titer, compared to the native form.

Another object of the present disclosure is to provide an insulin analog conjugate that is prepared by linking the insulin analog to the carrier.

Still another object of the present disclosure is to provide a long-acting insulin formulation including the insulin analog conjugate.

Still another object of the present disclosure is to provide a method for preparing the insulin analog conjugate.

Still another object of the present disclosure is to provide a method for increasing in vivo half-life using the insulin analog or the insulin analog conjugate prepared by linking the insulin analog to the carrier.

Also disclosed is a method for treating insulin-related diseases, including the step of administering the insulin analog or the insulin analog conjugate to a subject in need thereof.

### [Technical Solution]

The present invention is as defined in the claims. The present disclosure also describes related matter as set out below.

In one case to achieve the above objects, the present disclosure provides an insulin analog having a reduced insulin titer, compared to the native form, in which an amino acid of B chain or A chain is modified.

In one specific instance, the present disclosure provides an insulin analog having a reduced insulin receptor binding affinity.

In another specific instance, the present disclosure provides a non-native insulin analog, in which one amino acid selected from the group consisting of 8^{th} amino acid, 23^{th} amino acid, 24^{th} amino acid, and 25^{th} amino acid of B chain and 1^{th} amino acid, 2^{th} amino acid, and 19^{th} amino acid of A chain is substituted with alanine, or in which 14^{th} amino acid of A chain is substituted with glutamic acid or asparagine in the insulin analog according to the present disclosure.

In still another specific instance, the present disclosure provides an insulin analog, in which the insulin analog according to the present disclosure is selected from the group consisting of SEQ ID NOs. 20, 22, 24, 26, 28, 30, 32, 34 and 36.

In another case, the present disclosure provides an insulin analog conjugate that is prepared by linking the above described insulin analog to a carrier capable of prolonging half-life.

In one specific instance, the present disclosure provides an insulin analog conjugate, in which the insulin analog conjugate is prepared by linking (i) the above described insulin analog and (ii) an immunoglobulin Fc region via (iii) a peptide linker or a non-peptidyl linker selected from the group consisting of polyethylene glycol, polypropylene glycol, copolymers of ethylene glycol-propylene glycol, polyoxyethylated polyols, polyvinyl alcohols, polysaccharides, dextran, polyvinyl ethyl ether, biodegradable polymers, lipid polymers, chitins, hyaluronic acid, and combination thereof.

In still another case, the present disclosure provides a long-acting insulin formulation including the above described insulin analog conjugate, in which in vivo duration and stability are increased.

In one specific instance, the present disclosure provides a long-acting formulation that is used for the treatment of diabetes.

In another instance, the present disclosure provides a method for preparing the above described insulin analog conjugate.

In still another specific instance, the present disclosure provides a method for increasing in vivo half-life using the insulin analog or the insulin analog conjugate that is prepared by linking the insulin analog and the carrier.

Also disclosed is a method for treating insulin-related diseases, including the step of administering the insulin analog or the insulin analog conjugate to a subject in need thereof.

### [Advantageous Effects]

A non-native insulin analog of the present disclosure has a reduced insulin titer and a reduced insulin receptor binding affinity, compared to the native form, and thus avoids in vivo clearance mechanisms. Therefore, the insulin analog has increased blood half-life in vivo, and an insulin analog-immunoglobulin Fc conjugate prepared by using the same shows remarkably increased blood half-life, thereby improving convenience of patients in need of insulin administration.

### [Description of Drawings]

FIG. 1 shows the result of analyzing purity of an insulin analog by protein electrophoresis, which is the result of the representative insulin analog, Analog No. 7 (Lane 1: size marker, Lane 2: native insulin, Lane 3: insulin analog (No. 7) ;
FIG. 2 shows the result of analyzing purity of an insulin analog by high pressure chromatography, which is the result of the representative insulin analog, Analog No. 7 ((A) RP-HPLC, (B) SE-HPLC);
FIG. 3 shows the result of peptide mapping of an insulin analog, which is the result of the representative insulin analog, Analog No. 7 ((A) native insulin, (B) insulin analog (No. 7)) ;
FIG. 4 shows the result of analyzing purity of an insulin analog-immunoglobulin Fc conjugate by protein electrophoresis, which is the result of the representative insulin analog, Analog No. 7 (Lane 1: size marker, Lane 2: insulin analog (No. 7)-immunoglobulin Fc conjugate);
FIG. 5 shows the result of analyzing purity of an insulin analog-immunoglobulin Fc conjugate by high pressure chromatography, which is the result of the representative insulin analog, Analog No. 7 ((A) RP-HPLC, (B) SE-HPLC, (C) IE-HPLC); and

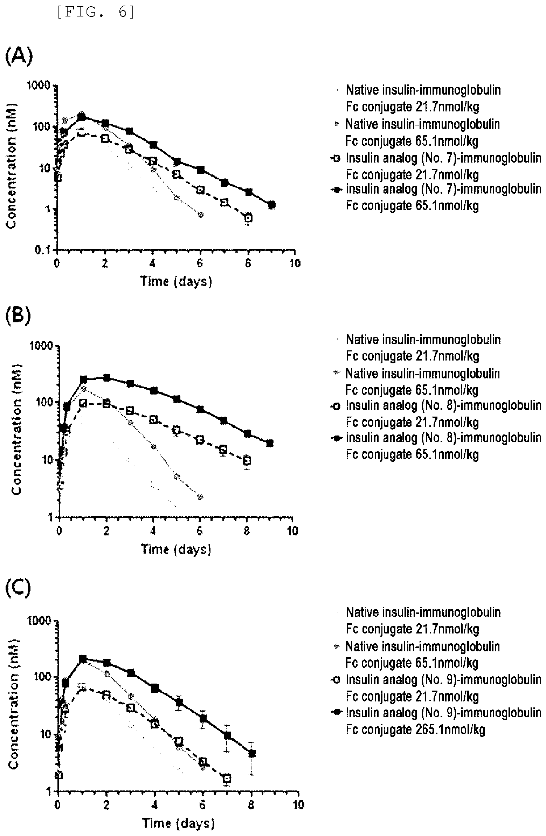

FIG. 6 shows the result of analyzing pharmacokinetics of native insulin-immunoglobulin Fc conjugate and insulin analog-immunoglobulin Fc conjugate in normal rats, which is the result of the representative insulin analog, Analog No. 7 (○: native insulin-immunoglobulin Fc conjugate (21.7 nmol/kg), ●: native insulin-immunoglobulin Fc conjugate (65.1 nmol/kg), □: insulin analog-immunoglobulin Fc conjugate (21.7 nmol/kg), ■: insulin analog-immunoglobulin Fc conjugate (65.1 nmol/kg). (A) native insulin-immunoglobulin Fc conjugate and insulin analog (No. 7)-immunoglobulin Fc conjugate, (B) native insulin-immunoglobulin Fc conjugate and insulin analog (No. 8)-immunoglobulin Fc conjugate, (C) native insulin-immunoglobulin Fc conjugate and insulin analog (No. 9)-immunoglobulin Fc conjugate).

### [Best Mode]

The present disclosure relates to an insulin analog having a reduced in-vitro titer. This insulin analog is characterized in that it has the non-native insulin sequence and therefore, has a reduced insulin receptor binding affinity, compared to the native insulin, and consequently, receptor-mediated clearance is remarkably reduced by increased dissociation constant, resulting in an increase in blood half-life.

As used herein, the term "insulin analog" includes various analogs having reduced insulin titer, compared to the native form.

The insulin analog may be an insulin analog having reduced insulin titer, compared to the native form, in which an amino acid of B chain or A chain of insulin is modified. The amino acid sequences of the native insulin are as follows.
- A chain:
   Gly-Ile-Val-Glu-Gln-Cys-Cys-Thr-Ser-Ile-Cys-Ser-Leu-Tyr-Gln-Leu-Glu-Asn-Tyr-Cys-Asn (SEQ ID NO. 37)
- B chain:
   Phe-Val-Asn-Gln-His-Leu-Cys-Gly-Ser-His-Leu-Val-Glu-Ala-Leu-Tyr-Leu-Val-Cys-Gly-Glu-Arg-Gly-Phe-Phe-Tyr-Thr-Pro-Lys-Thr (SEQ ID NO. 38)

The insulin analog used in Examples of the present disclosure is an insulin analog prepared by a genetic recombination technique. However, the present disclosure is not limited thereto, but includes all insulins having reduced in-vitro titer. Preferably, the insulin analog may include inverted insulins, insulin variants, insulin fragments or the like, and the preparation method may include a solid phase method as well as a genetic recombination technique, but is not limited thereto.

The insulin analog is a peptide retaining a function of controlling blood glucose in the body, which is identical to that of insulin, and this peptide includes insulin agonists, derivatives, fragments, variants thereof or the like.

The insulin agonist of the present disclosure refers to a substance which is bound to the in vivo receptor of insulin to exhibit the same biological activities as insulin, regardless of the structure of insulin.

The insulin analog of the present disclosure denotes a peptide which shows a sequence homology of at least 80% in an amino acid sequence as compared to A chain or B chain of the native insulin, has some groups of amino acid residues altered in the form of chemical substitution (e.g., alpha-methylation, alpha-hydroxylation), removal (e.g., deamination) or modification (e.g., N-methylation), and has a function of controlling blood glucose in the body. With respect to the objects of the present disclosure, the insulin analog is an insulin analog having a reduced insulin receptor binding affinity, compared to the native form, and insulin analogs having a reduced insulin titer compared to the native form are included without limitation.

As long as the insulin analog is able to exhibit low receptor-mediated internalization or receptor-mediated clearance, its type and size are not particularly limited. An insulin analog, of which major in vivo clearance mechanism is the receptor-mediated internalization or receptor-mediated clearance, is suitable for the objects of the present disclosure.

The insulin fragment of the present disclosure denotes the type of insulin in which one or more amino acids are added or deleted, and the added amino acids may be non-native amino acids (e.g., D-type amino acid). Such insulin fragments retain the function of controlling blood glucose in the body.

The insulin variant of the present disclosure denotes a peptide which differs from insulin in one or more amino acid sequences, and retains the function of controlling blood glucose in the body.

The respective methods for preparation of insulin agonists, derivatives, fragments and variants of the present disclosure can be used independently or in combination. For example, peptides of which one or more amino acid sequences differ from those of insulin and which have deamination at the amino-terminal amino acid residue and also have the function of controlling blood glucose in the body are included in the present disclosure.

Specifically, the insulin analog may be an insulin analog in which one or more amino acids selected from the group consisting of 8^{th} amino acid, 23^{th} amino acid, 24^{th} amino acid, and 25^{th} amino acid of B chain and 1^{th} amino acid, 2^{th} amino acid, 14^{th} amino acid and 19^{th} amino acid of A chain are substituted with other amino acid, and preferably, in which one or more amino acids selected from the group consisting of 8^{th} amino acid, 23^{th} amino acid, 24^{th} amino acid, and 25^{th} amino acid of B chain and 1^{th} amino acid, 2^{th} amino acid, and 19^{th} amino acid of A chain are substituted with alanine or in which 14^{th} amino acid of A chain is substituted with glutamic acid or asparagine. In addition, the insulin analog may be selected from the group consisting of SEQ ID NOs. 20, 22, 24, 26, 28, 30, 32, 34 and 36, but may include any insulin analog having a reduced insulin receptor binding affinity without limitation.

According to one instance of the present disclosure, the insulin analogs of SEQ ID NOs. 20, 22, 24, 26, 28, 30, 32, 34 and 36, in particular, the representative insulin analogs, 7, 8, and 9 (SEQ ID NOs. 32, 34, and 36) were found to have reduced insulin receptor binding affinity in vitro, compared to the native form (Table 4).

In another case, the present disclosure provides an insulin analog conjugate that is prepared by linking the insulin analog and a carrier.

As used herein, the term "carrier" denotes a substance capable of increasing in vivo half-life of the linked insulin analog. The insulin analog according to the present disclosure is characterized in that it has a remarkably reduced insulin receptor binding affinity, compared to the native form, and avoids receptor-mediated clearance or renal clearance. Therefore, if a carrier known to increase in vivo half-life when linked to the known various physiologically active polypeptides is linked with the insulin analog, it is apparent that in vivo half-life can be improved and the resulting conjugate can be used as a long-acting formulation.

For example, because half-life improvement is the first priority, the carrier to be linked with the novel insulin having a reduced titer is not limited to the immunoglobulin Fc region. The carrier includes a biocompatible material that is able to prolong in vivo half-life by linking it with any one biocompatible material, capable of reducing renal clearance, selected from the group consisting of various polymers (e.g., polyethylene glycol and fatty acid, albumin and fragments thereof, particular amino acid sequence, etc.), albumin and fragments thereof, albumin-binding materials, and polymers of repeating units of particular amino acid sequence, antibody, antibody fragments, FcRn-binding materials, in vivo connective tissue or derivatives thereof, nucleotide, fibronectin, transferrin, saccharide, and polymers, but is not limited thereto. In addition, the method for linking the biocompatible material capable of prolonging in vivo half-life to the insulin analog having a reduced titer includes genetic recombination, in vitro conjugation or the like. Examples of the biocompatible material may include an FcRn-binding material, fatty acid, polyethylene glycol, an amino acid fragment, or albumin. The FcRn-binding material may be an immunoglobulin Fc region.

The insulin analog and the biocompatible material as the carrier may be linked to each other via a peptide or a non-peptidyl polymer as a linker.

The insulin conjugate may be an insulin analog conjugate that is prepared by linking (i) the insulin analog and (ii) the immunoglobulin Fc region via (iii) a peptide linker or a non-peptidyl linker selected from the group consisting of polyethylene glycol, polypropylene glycol, copolymers of ethylene glycol-propylene glycol, polyoxyethylated polyol, polyvinyl alcohol, polysaccharides, dextran, polyvinyl ethyl ether, biodegradable polymer, lipid polymers, chitins, hyaluronic acid and combination thereof.

In one specific instance of the insulin analog conjugate of the present disclosure, a non-peptidyl polymer as a linker is linked to the amino terminus of B chain of the insulin analog. In another specific instance of the conjugate of the present disclosure, a non-peptidyl polymer as a linker is linked to the residue of B chain of the insulin analog. The modification in A chain of insulin leads to a reduction in the activity and safety. In these instances, therefore, the non-peptidyl polymer as a linker is linked to B chain of insulin, thereby maintaining insulin activity and improving safety.

As used herein, the term "activity" means the ability of insulin to bind to the insulin receptor, and means that insulin binds to its receptor to exhibit its action. Such binding of the non-peptidyl polymer to the amino terminus of B chain of insulin of the present disclosure can be achieved by pH control, and the preferred pH range is 4.5 to 7.5.

As used herein, the term "N-terminus" can be used interchangeably with "N-terminal region".

In one specific Example, the present authors prepared an insulin analog-PEG-immunoglobulin Fc conjugate by linking PEG to the N-terminus of an immunoglobulin Fc region, and selectively coupling the N-terminus of B chain of insulin thereto. The serum half-life of this insulin analog-PEG-immunoglobulin Fc conjugate was increased, compared to non-conjugate, and it showed a hypoglycemic effect in disease animal models. Therefore, it is apparent that a new long-acting insulin formulation maintaining in vivo activity can be prepared.

The immunoglobulin Fc region is safe for use as a drug carrier because it is a biodegradable polypeptide that is in vivo metabolized. Also, the immunoglobulin Fc region has a relatively low molecular weight, as compared to the whole immunoglobulin molecules, and thus, it is advantageous in terms of preparation, purification and yield of the conjugate. The immunoglobulin Fc region does not contain a Fab fragment, which is highly non-homogenous due to different amino acid sequences according to the antibody subclasses, and thus it can be expected that the immunoglobulin Fc region may greatly increase the homogeneity of substances and be less antigenic in blood.

As used herein, the term "immunoglobulin Fc region" refers to a protein that contains the heavy-chain constant region 2 (CH2) and the heavy-chain constant region 3 (CH3) of an immunoglobulin, excluding the variable regions of the heavy and light chains, the heavy-chain constant region 1 (CH1) and the light-chain constant region 1 (CL1) of the immunoglobulin. It may further include a hinge region at the heavy-chain constant region. Also, the immunoglobulin Fc region of the present disclosure may contain a part or all of the Fc region including the heavy-chain constant region 1 (CH1) and/or the light-chain constant region 1 (CL1), except for the variable regions of the heavy and light chains of the immunoglobulin, as long as it has an effect substantially similar to or better than that of the native form. Also, it may be a fragment having a deletion in a relatively long portion of the amino acid sequence of CH2 and/or CH3.

That is, the immunoglobulin Fc region of the present disclosure may include 1) a CH1 domain, a CH2 domain, a CH3 domain and a CH4 domain, 2) a CH1 domain and a CH2 domain, 3) a CH1 domain and a CH3 domain, 4) a CH2 domain and a CH3 domain, 5) a combination of one or more domains and an immunoglobulin hinge region (or a portion of the hinge region), and 6) a dimer of each domain of the heavy-chain constant regions and the light-chain constant region.

Further, the immunoglobulin Fc region of the present disclosure includes a sequence derivative (mutant) thereof as well as a native amino acid sequence. An amino acid sequence derivative has a sequence that is different from the native amino acid sequence due to a deletion, an insertion, a non-conservative or conservative substitution or combinations thereof of one or more amino acid residues. For example, in an IgG Fc, amino acid residues known to be important in binding, at positions 214 to 238, 297 to 299, 318 to 322, or 327 to 331, may be used as a suitable target for modification.

In addition, other various derivatives are possible, including derivatives having a deletion of a region capable of forming a disulfide bond, a deletion of several amino acid residues at the N-terminus of a native Fc form, or an addition of methionine residue to the N-terminus of a native Fc form. Furthermore, to remove effector functions, a deletion may occur in a complement-binding site, such as a C1q-binding site and an ADCC (antibody dependent cell mediated cytotoxicity) site. Techniques of preparing such sequence derivatives of the immunoglobulin Fc region are disclosed in WO 97/34631 and WO 96/32478.

Amino acid exchanges in proteins and peptides, which do not generally alter the activity of molecules, are known in the art (H.Neurath, R.L.Hill, The Proteins, Academic Press, New York, 1979). The most commonly occurring exchanges are Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, Asp/Gly, in both directions.

The Fc region, if desired, may be modified by phosphorylation, sulfation, acrylation, glycosylation, methylation, farnesylation, acetylation, amidation or the like.

The aforementioned Fc derivatives are derivatives that have a biological activity identical to that of the Fc region of the present disclosure or improved structural stability against heat, pH, or the like.

In addition, these Fc regions may be obtained from native forms isolated from humans and other animals including cows, goats, swine, mice, rabbits, hamsters, rats and guinea pigs, or may be recombinants or derivatives thereof, obtained from transformed animal cells or microorganisms. Here, they may be obtained from a native immunoglobulin by isolating whole immunoglobulins from human or animal organisms and treating them with a proteolytic enzyme. Papain digests the native immunoglobulin into Fab and Fc regions, and pepsin treatment results in the production of pF'c and F(ab)₂. These fragments may be subjected to size-exclusion chromatography to isolate Fc or pF'c.

Preferably, a human-derived Fc region is a recombinant immunoglobulin Fc region that is obtained from a microorganism.

In addition, the immunoglobulin Fc region may be in the form of having native sugar chains, increased sugar chains compared to a native form or decreased sugar chains compared to the native form, or maybe in a deglycosylated form. The increase, decrease or removal of the immunoglobulin Fc sugar chains may be achieved by methods common in the art, such as a chemical method, an enzymatic method and a genetic engineering method using a microorganism. Here, the removal of sugar chains from an Fc region results in a sharp decrease in binding affinity to the complement (clq) and a decrease or loss in antibody-dependent cell-mediated cytotoxicity or complement-dependent cytotoxicity, thereby not inducing unnecessary immune responses in-vivo. In this regard, an immunoglobulin Fc region in a deglycosylated or aglycosylated form may be more suitable to the object of the present disclosure as a drug carrier.

The term "deglycosylation", as used herein, means to enzymatically remove sugar moieties from an Fc region, and the term "aglycosylation" means that an Fc region is produced in an unglycosylated form by a prokaryote, preferably, E. coli.

On the other hand, the immunoglobulin Fc region may be derived from humans or other animals including cows, goats, swine, mice, rabbits, hamsters, rats and guinea pigs, and preferably humans. In addition, the immunoglobulin Fc region may be an Fc region that is derived from IgG, IgA, IgD, IgE and IgM, or that is made by combinations thereof or hybrids thereof. Preferably, it is derived from IgG or IgM, which is among the most abundant proteins in human blood, and most preferably, from IgG which is known to enhance the half-lives of ligand-binding proteins.

On the other hand, the term "combination", as used herein, means that polypeptides encoding single-chain immunoglobulin Fc regions of the same origin are linked to a single-chain polypeptide of a different origin to form a dimer or multimer. That is, a dimer or multimer may be formed from two or more fragments selected from the group consisting of IgG Fc, IgA Fc, IgM Fc, IgD Fc and IgE Fc fragments.

The term "hybrid", as used herein, means that sequences encoding two or more immunoglobulin Fc regions of different origin are present in a single-chain immunoglobulin Fc region. In the present disclosire, various types of hybrids are possible. That is, domain hybrids may be composed of one to four domains selected from the group consisting of CH1, CH2, CH3 and CH4 of IgG Fc, IgM Fc, IgA Fc, IgE Fc and IgD Fc, and may include the hinge region.

On the other hand, IgG is divided into IgG1, IgG2, IgG3 and IgG4 subclasses, and the present disclosure includes combinations and hybrids thereof. Preferred are IgG2 and IgG4 subclasses, and most preferred is the Fc region of IgG4 rarely having effector functions such as CDC (complement dependent cytotoxicity). That is, as the drug carrier of the present disclosure, the most preferable immunoglobulin Fc region is a human IgG4-derived non-glycosylated Fc region. The human-derived Fc region is more preferable than a non-human derived Fc region which may act as an antigen in the human body and cause undesirable immune responses such as the production of a new antibody against the antigen.

In the specific instance of the insulin analog conjugate, both ends of the non-peptidyl polymer may be linked to the N-terminus of the immunoglobulin Fc region and the amine group of the N-terminus of B chain of the insulin analog or the ε-amino group or the thiol group of the internal lysine residue of B chain, respectively.

The Fc region-linker-insulin analog of the present disclosure is made at various molar ratios. That is, the number of the Fc fragment and/or linker linked to a single insulin analog is not limited.

In addition, the linkage of the Fc region, a certain linker, and the insulin analog of the present disclosure may include all types of covalent bonds and all types of noncovalent bonds such as hydrogen bonds, ionic interactions, van der Waals forces and hydrophobic interactions when the Fc region and the insulin analog are expressed as a fusion protein by genetic recombination. However, with respect to the physiological activity of the insulin analog, the linkage is preferably made by covalent bonds, but is not limited thereto.

On the other hand, the Fc region of the present disclosure, a certain linker and the insulin analog may be linked to each other at an N-terminus or C-terminus, and preferably at a free group, and especially, a covalent bond may be formed at an amino terminal end, an amino acid residue of lysine, an amino acid residue of histidine, or a free cysteine residue.

In addition, the linkage of the Fc region of the present disclosure, a certain linker, and the insulin analog may be made in a certain direction. That is, the linker may be linked to the N-terminus, the C-terminus or a free group of the immunoglobulin Fc region, and may also be linked to the N-terminus, the C-terminus or a free group of the insulin analog. The non-peptidyl linker may be linked to the N-terminal amine group of the immunoglobulin fragment, and is not limited to any of the lysine residue or cysteine residue of the immunoglobulin fragment sequence.

Further, in the specific instance of the insulin analog conjugate, the end of the non-peptidyl polymer may be linked to the internal amino acid residue or free reactive group capable of binding to the reactive group at the end of the non-peptidyl polymer, in addition to the N-terminus of the immunoglobulin Fc region, but is not limited thereto.

In the present disclosure, the non-peptidyl polymer means a biocompatible polymer including two or more repeating units linked to each other, in which the repeating units are linked by any covalent bond excluding the peptide bond. Such non-peptidyl polymer may have two ends or three ends.

The non-peptidyl polymer which can be used in the present disclosure may be selected from the group consisting of polyethylene glycol, polypropylene glycol, copolymers of ethylene glycol and propylene glycol, polyoxyethylated polyols, polyvinyl alcohol, polysaccharides, dextran, polyvinyl ethyl ether, biodegradable polymers such as PLA (poly(lactic acid)) and PLGA (polylactic-glycolic acid), lipid polymers, chitins, hyaluronic acid, and combinations thereof, and preferably, polyethylene glycol. The derivatives thereof well known in the art and being easily prepared within the skill of the art are also included in the scope of the present disclosure.

The peptide linker which is used in the fusion protein obtained by a conventional inframe fusion method has drawbacks in that it is easily in-vivo cleaved by a proteolytic enzyme, and thus a sufficient effect of increasing the blood half-life of the active drug by a carrier cannot be obtained as expected. In the present disclosure, however, the conjugate can be prepared using the non-peptidyl linker as well as the peptide linker. In the non-peptidyl linker, the polymer having resistance to the proteolytic enzyme can be used to maintain the blood half-life of the peptide being similar to that of the carrier. Therefore, any non-peptidyl polymer can be used without limitation, as long as it is a polymer having the aforementioned function, that is, a polymer having resistance to the in-vivo proteolytic enzyme. The non-peptidyl polymer has a molecular weight ranging from 1 to 100 kDa, and preferably, ranging from 1 to 20 kDa.

The non-peptidyl polymer of the present disclosure, linked to the immunoglobulin Fc region, may be one polymer or a combination of different types of polymers.

The non-peptidyl polymer used in the present disclosure has a reactive group capable of binding to the immunoglobulin Fc region and the protein drug.

The non-peptidyl polymer has a reactive group at both ends, which is preferably selected from the group consisting of a reactive aldehyde group, a propionaldehyde group, a butyraldehyde group, a maleimide group and a succinimide derivative. The succinimide derivative may be succinimidyl propionate, hydroxy succinimidyl, succinimidyl carboxymethyl, or succinimidyl carbonate. In particular, when the non-peptidyl polymer has a reactive aldehyde group at both ends thereof, it is effective in linking at both ends with a physiologically active polypeptide and an immunoglobulin with minimal non-specific reactions. A final product generated by reductive alkylation by an aldehyde bond is much more stable than that linked by an amide bond. The aldehyde reactive group selectively binds to an N-terminus at a low pH, and binds to a lysine residue to form a covalent bond at a high pH, such as pH 9.0.

The reactive groups at both ends of the non-peptidyl polymer may be the same as or different from each other. For example, the non-peptide polymer may possess a maleimide group at one end, and an aldehyde group, a propionaldehyde group or a butyraldehyde group at the other end. When a polyethylene glycol having a reactive hydroxy group at both ends thereof is used as the non-peptidyl polymer, the hydroxy group may be activated to various reactive groups by known chemical reactions, or a polyethylene glycol having a commercially available modified reactive group may be used so as to prepare the single chain insulin analog conjugate of the present disclosure.

The insulin analog conjugate of the present disclosure maintains in vivo activities of the conventional insulin such as energy metabolism and sugar metabolism, and also increases blood half-life of the insulin analog and markedly increases duration of in-vivo efficacy of the peptide, and therefore, the conjugate is useful in the treatment of diabetes.

In one Example of the present disclosure, it was confirmed that the insulin analog having a reduced insulin receptor binding affinity exhibits much higher in vivo half-life than the native insulin conjugate, when linked to the carrier capable of prolonging in vivo half-life (FIG. 6).

In another case, the present disclosure provides a long-acting insulin formulation including the insulin analog conjugate. The long-acting insulin formulation may be a long-acting insulin formulation having increased in vivo duration and stability. The long-acting formulation may be a pharmaceutical composition for the treatment of diabetes.

The pharmaceutical composition including the conjugate of the present disclosure may include pharmaceutically acceptable carriers. For oral administration, the pharmaceutically acceptable carrier may include a binder, a lubricant, a disintegrator, an excipient, a solubilizer, a dispersing agent, a stabilizer, a suspending agent, a coloring agent, a perfume or the like. For injectable preparations, the pharmaceutically acceptable carrier may include a buffering agent, a preserving agent, an analgesic, a solubilizer, an isotonic agent, and a stabilizer. For preparations for topical administration, the pharmaceutically acceptable carrier may include a base, an excipient, a lubricant, a preserving agent or the like. The pharmaceutical composition of the present disclosure may be formulated into a variety of dosage forms in combination with the aforementioned pharmaceutically acceptable carriers. For example, for oral administration, the pharmaceutical composition may be formulated into tablets, troches, capsules, elixirs, suspensions, syrups or wafers. For injectable preparations, the pharmaceutical composition may be formulated into single-dose ampule or multidose container. The pharmaceutical composition may be also formulated into solutions, suspensions, tablets, pills, capsules and sustained release preparations.

On the other hand, examples of carriers, excipients and diluents suitable for formulation include lactose, dextrose, sucrose, sorbitol, mannitol, xylitol, erythritol, maltitol, starch, acacia, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, mineral oils or the like.

In addition, the pharmaceutical formulations may further include fillers, anti-coagulating agents, lubricants, humectants, perfumes, antiseptics or the like.

Also disclosed herein is a method for treating insulin-related diseases, including administering the insulin analog or the insulin analog conjugate to a subject in need thereof.

The conjugate according to the present disclosure is useful in the treatment of diabetes, and therefore, this disease can be treated by administering the pharmaceutical composition including the same.

The term "administration", as used herein, means introduction of a predetermined substance into a patient by a certain suitable method. The conjugate of the present disclosure may be administered via any of the common routes, as long as it is able to reach a desired tissue. Intraperitoneal, intravenous, intramuscular, subcutaneous, intradermal, oral, topical, intranasal, intrapulmonary and intrarectal administration can be performed, but the present disclosure is not limited thereto. However, since peptides are digested upon oral administration, active ingredients of a composition for oral administration should be coated or formulated for protection against degradation in the stomach. Preferably, the present composition may be administered in an injectable form. In addition, the pharmaceutical composition may be administered using a certain apparatus capable of transporting the active ingredients into a target cell.

Further, the pharmaceutical composition of the present disclosure may be determined by several related factors including the types of diseases to be treated, administration routes, the patient's age, gender, weight and severity of the illness, as well as by the types of the drug as an active component. Since the pharmaceutical composition of the present disclosure has excellent in vivo duration and titer, it has an advantage of greatly reducing administration frequency of the pharmaceutical formulation of the present disclosure.

In still another case, the present disclosure provides a method for preparing the insulin analog conjugate, including preparing the insulin analog; preparing the carrier; and linking the insulin analog and the carrier.

In still another case, the present disclosure provides a method for increasing in vivo half-life using the insulin analog or the insulin analog conjugate which is prepared by linking the insulin analog and the carrier.

### [Mode]

Hereinafter, the present disclosure will be described in more detail with reference to Examples. However, these Examples are for illustrative purposes only, and the disclosure is not intended to be limited by these Examples.

### Example 1: Preparation of single chain insulin analog-expressing vector

In order to prepare insulin analogs, each of them having a modified amino acid in A chain or B chain, using the native insulin-expressing vector as a template, forward and reverse oligonucleotides were synthesized (Table 2), and then PCR was carried out to amplify each analog gene.

In the following Table 1, amino acid sequences modified in A chain or B chain and analog names are given. That is, Analog 1 represents that 1^{st} glycine of A chain is substituted with alanine, and Analog 4 represents that 8^{th} glycine of B chain is substituted with alanine.

**[Table 1]**

| Analog | Modifed seqeunce |
|---|---|
| Analog 1 | A¹G → A |
| Analog 2 | A² → A |
| Analog 3 | A¹⁹Y → A |
| Analog 4 | B⁸G → A |
| Analog 5 | B²³G → A |
| Analog 6 | B²⁴F → A |
| Analog 7 | B²⁵F → A |
| Analog 8 | A¹⁴ Y → E |
| Analog 9 | A¹⁴ Y → N |

Primers for insulin analog amplification are given in the following Table 2.

**[Table 2]**

| **Analogs** | **Sequence** | **SEQ ID NO.** |
|---|---|---|
| Analog 1 | 5' GGGTCCCTGCAGAAGCGTGCGATTGTGGAACAATGCTGT 3' | SEQ ID NO.1 |
| | 5' ACAGCATTGTTCCACAATCGCACGCTTCTGCAGGGACCC 3' | SEQ ID NO.2 |
| Analog 2 | 5' TCCCTGCAGAAGCGTGGCGCGGTGGAACAATGCTGTACC 3' | SEQ ID NO.3 |
| | 5' GGTACAGCATTGTTCCACCGCGCCACGCTTCTGCAGGGA 3' | SEQ ID NO.4 |
| Analog 3 | 5' CTCTACCAGCTGGAAAACGCGTGTAACTGAGGATCCC 3' | SEQ ID NO.5 |
| | 5' GGTCCTCAGTTACACGCGTTTTCCAGCTGGTATAT 3' | SEQ ID NO.6 |
| Analog 4 | 5' VTTAACCAACACTTGTGTGCGTCACACCTGGTGGAAGCT 3' | SEQ ID NO.7 |
| | 5' AGCTTCCACCAGGTGTGACGCACACAAGTGTTGGTTAAC 3' | SEQ ID NO.8 |
| Analog 5 | 5' CTAGTGTGCGGGGAACGAGCGTTCTTCTACACACCCAAG 3' | SEQ ID NO.9 |
| | 5' CTTGGGTGTGTAGAAGAACGCTGTTCCCCGCACACATAG 3' | SEQ ID NO.10 |
| Analog 6 | 5' GTGTCGGGGAACGAGGCGCGTTCTACACACCCAAGACC 3' | SEQ ID NO.11 |
| | 5' GGTCTTGGGTGTGTAGAACGCGCCTCGTTTCCCCGCACAC 3' | SEQ ID NO.12 |
| Analog 7 | 5' TGCGGGGAACGAGGCTTCGCGCTACACACCCAAGACCCGC 3' | SEQ ID NO.13 |
| | 5' GCGGGTCTTGGGTGTGTACGCGAAGCCTCGTTCCCCGCA 3' | SEQ ID NO.14 |
| Analog 8 | 5'-CCAGCATCTGCTCCCTCGAACAGCTGGAGAACTACTG-3' | SEQ ID NO.15 |
| | 5'-Cagtagttctccagctgttcgagggagcagatgctgg 3' | SEQ ID NO.16 |
| Analog 9 | 5'-CAGCATCTGCTCCCTCAACCAGCTGGAGAACTAC 3' | SEQ 1D NO.17 |
| | 5' Gtagttctccagctggttgagggagcagatgctg3' | SEQ ID NO.18 |

PCR for insulin analog amplification was carried out under conditions of 95°C for 30 seconds, 55°C for 30 seconds, 68°C for 6 minutes for 18 cycles. The insulin analog fragments obtained under the conditions were inserted into pET22b vector to be expressed as intracellular inclusion bodies, and the resulting expression vectors were designated as pET22b-insulin analogs 1 to 9. The expression vectors contained nucleic acids encoding amino acid sequences of insulin analogs 1 to 9 under the control of T7 promoter, and insulin analog proteins were expressed as inclusion bodies in host cells.

DNA sequences and protein sequences of insulin analogs 1 to 9 are given in the following Table 3.

**[Table 3]**

| Analog | Sequence | | SEQ ID NO. |
|---|---|---|---|
| Analog 1 | DNA | 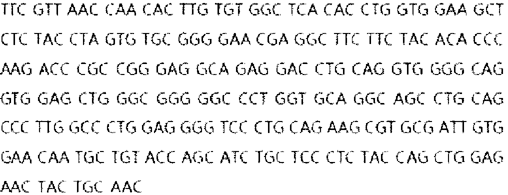 | 19 |
| | Protein | 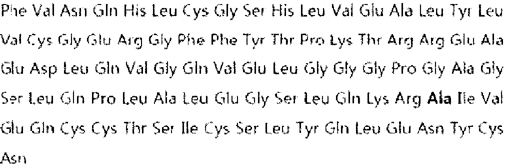 | 20 |
| Analog 2 | DNA | 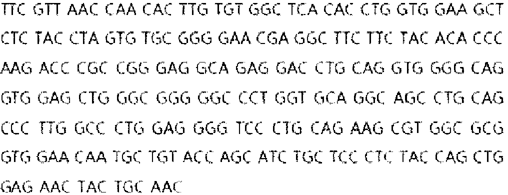 | 21 |
| | Protein | 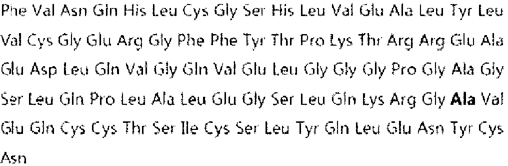 | 22 |
| Analog 3 | DNA | 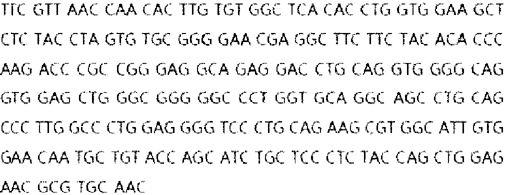 | 23 |
| | Protein | 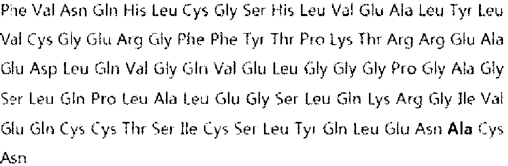 | 24 |
| Analog 4 | DNA | 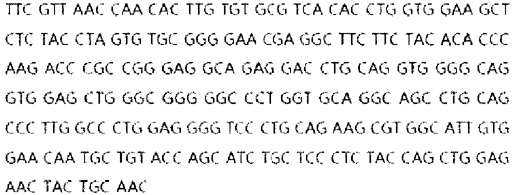 | 25 |
| | Protein | 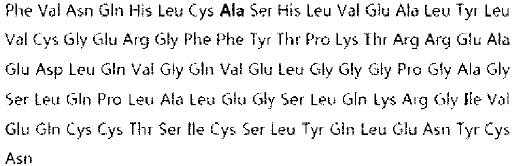 | 26 |
| Analog 5 | DNA | 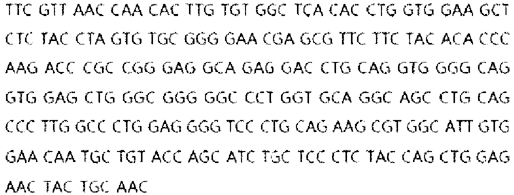 | 27 |
| | Protein | 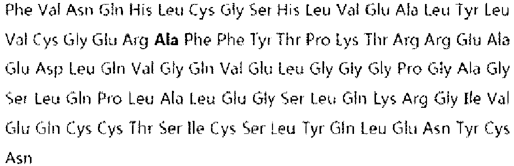 | 28 |
| Analog 6 | DNA | 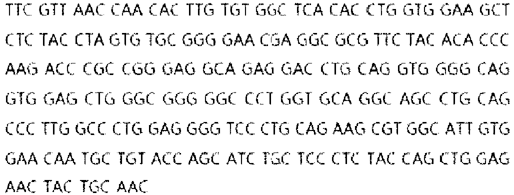 | 29 |
| | Protein | 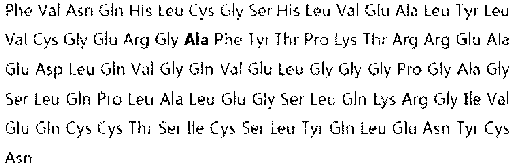 | 30 |
| Analog 7 | DNA | 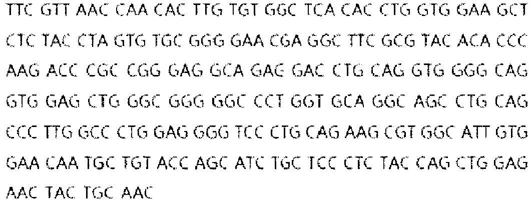 | 31 |
| | Protein | 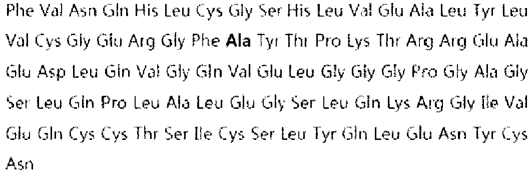 | 32 |
| Analog 8 | DNA | 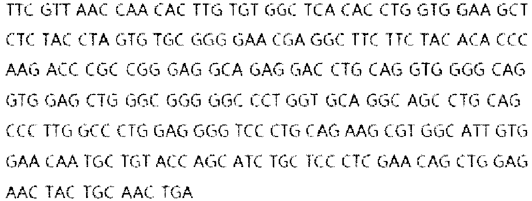 | 33 |
| | Protein | 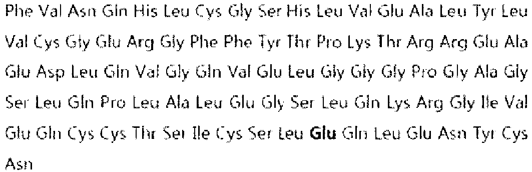 | 34 |
| Analog 9 | DNA | 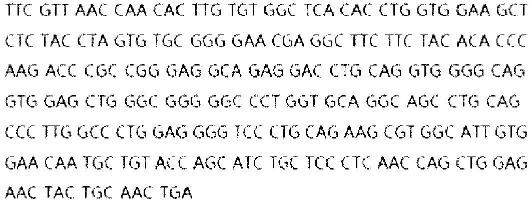 | 35 |
| | Protein | 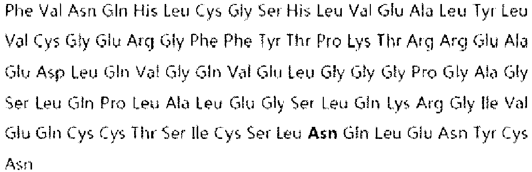 | 36 |

### Example 2: Expression of recombinant insulin analog fusion peptide

Expressions of recombinant insulin analogs were carried out under the control of T7 promoter. E.coli BL21-DE3 (E. coli B F-dcm ompT hsdS(rB-mB-) gal λDE3); Novagen) was transformed with each of the recombinant insulin analog-expressing vectors. Transformation was performed in accordance with the recommended protocol (Novagen). Single colonies transformed with each recombinant expression vector were collected and inoculated in 2X Luria Broth (LB) containing ampicillin (50 µg/ml) and cultured at 37°C for 15 hours. The recombinant strain culture broth and 2X LB medium containing 30% glycerol were mixed at a ratio of 1:1 (v/v). Each 1 ml was dispensed to a cryotube and stored at -140°C, which was used as a cell stock for production of the recombinant fusion protein.

To express the recombinant insulin analogs, 1 vial of each cell stock was thawed and inoculated in 500 ml of 2X Luria broth, and cultured with shaking at 37°C for 14∼16 hours. The cultivation was terminated, when OD600 reached 5.0 or higher. The culture broth was used as a seed culture broth. This seed culture broth was inoculated to a 50 L fermentor (MSJ-U2, B.E.MARUBISHI, Japan) containing 17 L of fermentation medium, and initial bath fermentation was started. The culture conditions were maintained at a temperature of 37°C, an air flow rate of 20 L/min (1 vvm), an agitation speed of 500 rpm, and at pH 6.70 by using a 30% ammonia solution. Fermentation was carried out in fed-batch mode by adding a feeding solution, when nutrients were depleted in the culture broth. Growth of the strain was monitored by OD value. IPTG was introduced in a final concentration of 500 µM, when OD value was above 100. After introduction, the cultivation was further carried out for about 23∼25 hours. After terminating the cultivation, the recombinant strains were harvested by centrifugation and stored at -80°C until use.

### Example 3: Recovery and Refolding of recombinant insulin analog

In order to change the recombinant insulin analogs expressed in Example 2 into soluble forms, cells were disrupted, followed by refolding. 100 g (wet weight) of the cell pellet was re-suspended in 1 L lysis buffer (50 mM Tris-HCl (pH 9.0), 1 mM EDTA (pH 8.0), 0.2 M NaCl and 0.5% Triton X-100). The cells were disrupted using a microfluidizer processor M-110EH (AC Technology Corp. Model M1475C) at an operating pressure of 15,000 psi. The cell lysate thus disrupted was centrifuged at 7,000 rpm and 4°C for 20 minutes. The supernatant was discarded and the pellet was re-suspended in 3 L washing buffer (0.5% Triton X-100 and 50 mM Tris-HCl (pH 8.0), 0.2 M NaCl, 1 mM EDTA). After centrifugation at 7,000 rpm and 4°C for 20 minutes, the cell pellet was re-suspended in distilled water, followed by centrifugation in the same manner. The pellet thus obtained was re-suspended in 400 ml of buffer (1 M Glycine, 3.78 g Cysteine-HCl, pH 10.6) and stirred at room temperature for 1 hour. To recover the recombinant insulin analog thus re-suspended, 400 mL of 8M urea was added and stirred at 40°C for 1 hour. For refolding of the solubilized recombinant insulin analogs, centrifugation was carried out at 7,000 rpm and 4°C for 30 minutes, and the supernatant was obtained. 2 L of distilled water was added thereto using a peristaltic pump at a flow rate of 1000 ml/hr while stirring at 4°C for 16 hours.

### Example 4: Cation binding chromatography purification

The sample refolded was loaded onto a Source S (GE healthcare) column equilibrated with 20 mM sodium citrate (pH 2.0) buffer containing 45% ethanol, and then the insulin analog proteins were eluted in 10 column volumes with a linear gradient from 0% to 100% 20 mM sodium citrate (pH 2.0) buffer containing 0.5 M potassium chloride and 45% ethanol.

### Example 5: Trypsin and Carboxypeptidase B treatment

Salts were removed from the eluted samples using a desalting column, and the buffer was exchanged with a buffer (10 mM Tris-HCl, pH 8.0). With respect to the obtained sample protein, trypsin corresponding to 1000 molar ratio and carboxypeptidase B corresponding to 2000 molar ratio were added, and then stirred at 16°C for 16 hours. To terminate the reaction, 1 M sodium citrate (pH 2.0) was used to reduce pH to 3.5.

### Example 6: Cation binding chromatography purification

The sample thus reacted was loaded onto a Source S (GE healthcare) column equilibrated with 20 mM sodium citrate (pH 2.0) buffer containing 45% ethanol, and then the insulin analog proteins were eluted in 10 column volumes with a linear gradient from 0% to 100% 20 mM sodium citrate (pH 2.0) buffer containing 0.5 M potassium chloride and 45% ethanol.

### Example 7: Anion binding chromatography purification

Salts were removed from the eluted sample using a desalting column, and the buffer was exchanged with a buffer (10 mM Tris-HCl, pH 7.5). In order to isolate a pure insulin analog from the sample obtained in Example 6, the sample was loaded onto an anion exchange column (Source Q: GE healthcare) equilibrated with 10 mM Tris (pH 7.5) buffer, and the insulin analog protein was eluted in 10 column volumes with a linear gradient from 0% to 100% 10 mM Tris (pH 7.5) buffer containing 0.5 M sodium chloride.

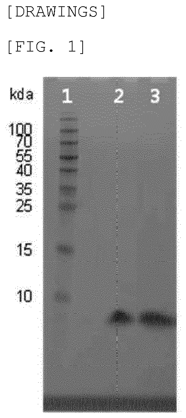

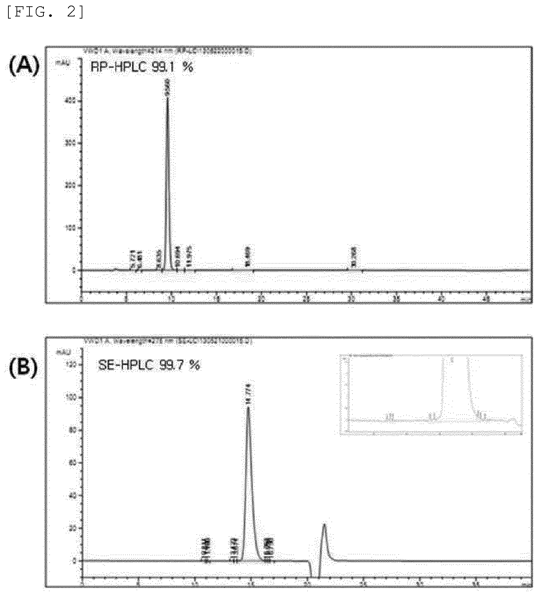

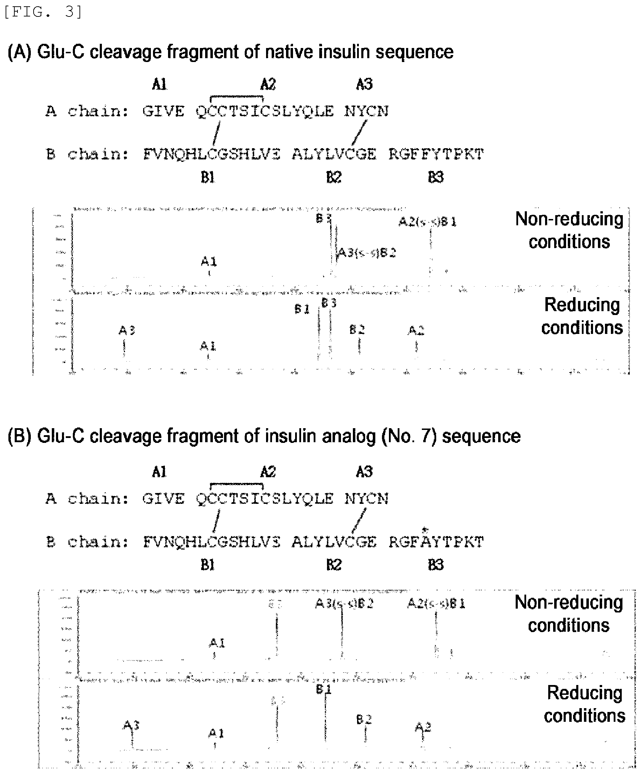

Purity of the insulin analog thus purified was analyzed by protein electrophoresis (SDS-PAGE, FIG. 1) and high pressure chromatography (HPLC) (FIG. 2), and modifications of amino acids were identified by peptide mapping (FIG. 3) and molecular weight analysis of each peak.

As a result, each insulin analog was found to have the desired modification in its amino acid sequence.

### Example 8: Preparation of insulin analog (No. 7)-immunoglobulin Fc conjugate

To pegylate the N-terminus of the beta chain of the insulin analog using 3.4K ALD2 PEG (NOF, Japan), the insulin analog and PEG were reacted at a molar ratio of 1:4 with an insulin analog concentration of 5 mg/ml at 4°C for about 2 hours. At this time, the reaction was performed in 50 mM sodium citrate at pH 6.0 and 45% isopropanol. 3.0 mM sodium cyanoborohydride was added as a reducing agent and was allowed to react. The reaction solution was purified with SP-HP (GE Healthcare, USA) column using a buffer containing sodium citrate (pH 3.0) and 45% ethanol, and KCl concentration gradient.

To prepare an insulin analog-immunoglobulin Fc fragment conjugate, the purified mono-PEGylated insulin analog and the immunoglobulin Fc fragment were reacted at a molar ratio of 1:1 to 1:2 and at 25°C for 13 hrs, with a total protein concentration of about 20 mg/ml. At this time, the reaction buffer conditions were 100 mM HEPES at pH 8.2, and 20 mM sodium cyanoborohydride as a reducing agent was added thereto. Therefore, PEG was bound to the N-terminus of the Fc fragment.

After the reaction was terminated, the reaction solution was loaded onto the Q HP (GE Healthcare, USA) column with Tris-HCl (pH 7.5) buffer and NaCl concentration gradient to separate and purify unreacted immunoglobulin Fc fragment and mono-PEGylated insulin analog.

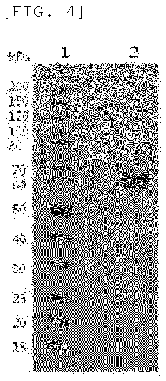

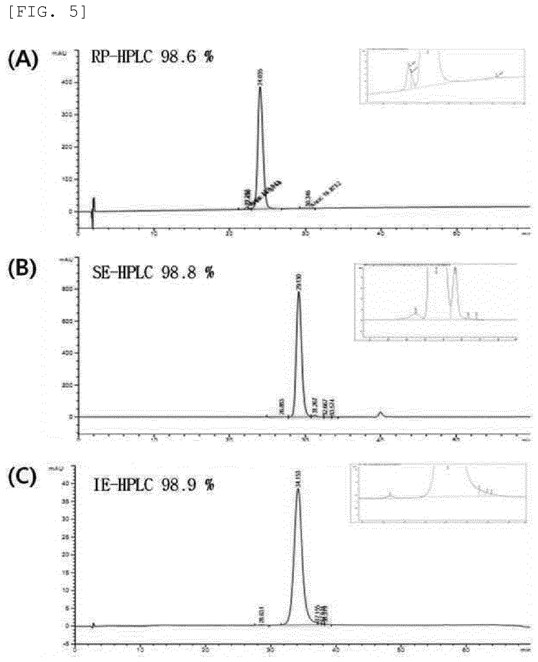

Thereafter, Source 15ISO (GE Healthcare, USA) was used as a secondary column to remove the remaining immunoglobulin Fc fragment and the conjugate, in which two or more insulin analogs were linked to the immunoglobulin Fc fragment, thereby obtaining the insulin analog-immunoglobulin Fc fragment conjugate. At this time, elution was carried out using a concentration gradient of ammonium sulfate containing Tris-HCl (pH 7.5), and the insulin analog-immunoglobulin Fc conjugate thus eluted was analyzed by protein electrophoresis (SDS-PAGE, FIG. 4) and high pressure chromatography (HPLC) (FIG. 5). As a result, the conjugate was found to have almost 99% purity.

### Example 9: Comparison of insulin receptor binding affinity between native insulin, insulin analog, native insulin-immunoglobulin Fc conjugate, and insulin analog-immunoglobulin Fc conjugate

In order to measure the insulin receptor binding affinity of the insulin analog-immunoglobulin Fc conjugate, Surface plasmon resonance (SPR, BIACORE 3000, GE healthcare) was used for analysis. Insulin receptors were immobilized on a CM5 chip by amine coupling, and 5 dilutions or more of native insulin, insulin analog, native insulin-immunoglobulin Fc conjugate, and insulin analog-immunoglobulin Fc conjugate were applied thereto, independently. Then, the insulin receptor binding affinity of each substance was examined. The binding affinity of each substance was calculated using BIAevaluation software. At this time, the model used was 1:1 Langmuir binding with baseline drift.

As a result, compared to human insulin, insulin analog (No. 6) showed receptor binding affinity of 14.8%, insulin analog (No. 7) showed receptor binding affinity of 9.9%, insulin analog (No. 8) showed receptor binding affinity of 57.1%, insulin analog (No. 9) showed receptor binding affinity of 78.8%, native insulin-immunoglobulin Fc conjugate showed receptor binding affinity of 3.7-5.9% depending on experimental runs, insulin analog (No. 6)-immunoglobulin Fc conjugate showed receptor binding affinity of 0.9% or less, insulin analog (No. 7)-immunoglobulin Fc conjugate showed receptor binding affinity of 1.9%, insulin analog (No. 8)-immunoglobulin Fc conjugate showed receptor binding affinity of 1.8%, and insulin analog (No. 9)-immunoglobulin Fc conjugate showed receptor binding affinity of 3.3% (Table 4). As such, it was observed that the insulin analogs of the present disclosure had reduced insulin receptor binding affinity, compared to the native insulin, and the insulin analog-immunoglobulin Fc conjugates also had remarkably reduced insulin receptor binding affinity.

**[Table 4]**

| **Comparison of insulin receptor binding affinity** | | | | |
|---|---|---|---|---|
| **Test No.** | **Substance name** | **kₐ (1/Ms, X10⁸)** | **K_{c} (1/s. X10⁻³)** | **K_{C} (nM)** |
| Test 1 | Native human insulin | 2.21 | 7.47 | 35.05 |
| | | (100%) | (100%) | (100%) |
| | Insulin analog (No. 6) | 0.28 | 6.60 | 237.0 |
| | | (12.6%) | (88.4%) | (14.8%) |
| Test 2 | Native human insulin | 2.29 | 10.1 | 46.1 |
| | | (100%) | (100%) | (100%) |
| | Native insulin-immunoglobulin Fc conjugate | 0.09 | 7.8 | 781.3 |
| | | (3.9%) | (77.2%) | (5.9%) |
| | Insulin analog (No. 6)-immunoglobulin Fc conjugate | 0.02 | 10. 1 | 5260.0 |
| | | (0.9%) | (100%) | (0.9%) |
| Test 3 | Native human insulin | 1.76 | 10.73 | 63.47 |
| | | (100%) | (100%) | (100%) |
| | Insulin analog (No. 7) | 0.14 | 8.34 | 642.0 |
| | | (7.8%) | (77.7%) | (9.9%) |
| | Native insulin-immunoglobulin Fc conjugate | 0.05 | 5.85 | 1236.67 |
| | | (2.7%) | (54.5%) | (5.1%) |
| | Insulin analog (No. 7)-immunoglobulin Fc conjugate | 0.02 | 7.20 | 3270.0 |
| | | (1.3%) | (67.1%) | (1.3%) |
| Test 4 | Native human insulin | 2.9 | 12.4 | 42.0 |
| | | (100%) | (100%) | (100%) |
| | Insulin analog (No. 8) | 1.78 | 12.9 | 73.4 |
| | | (60.0%) | (104.6%) | (57.1%) |
| | Native insulin-immunoglobulin Fc conjugate | 0.06 | 6.9 | 1140.0 |
| | | (2.1%) | (56.1%) | (3.7%) |
| | Insulin analog (No. 8)-immunoglobulin Fc conjugate | 0.03 | 6.4 | 2320.0 |
| | | (0.9%) | (51.5%) | (1.8%) |
| Test 5 | Native human insulin | 2.0 | 9.7 | 50.4 |
| | | (100%) | (100%) | (100%) |
| | Insulin analog (No. 9) | 1.85 | 11.9 | 64.0 |
| | | (92.5%) | (122.5%) | (78.8%) |
| | Native insulin-immunoglobulin Fc conjugate | 0.09 | 7.4 | 862.0 |
| | | (4.3%) | (76.5%) | (5.9%) |
| | Insulin analog (No. 9)-immunoglobulin Fc conjugate | 0.05 | 7.3 | 1536.7 |
| | | (2.4%) | (75.0%) | (3.3%) |

### Example 10: Comparison of in-vitro efficacy between native insulin-immunoglobulin Fc conjugate and insulin analog-immunoglobulin Fc conjugate

In order to evaluate in vitro efficacy of the insulin analog-immunoglobulin Fc conjugate, mouse-derived differentiated 3T3-L1 adipocytes were used to test glucose uptake or lipid synthesis. 3T3-L1 cells were sub-cultured in 10% NBCS (newborn calf serum)-containing DMEM (Dulbeco's Modified Eagle's Medium, Gibco, Cat.No, 12430) twice or three times a week, and maintained. 3T3-L1 cells were suspended in a differentiation medium (10% FBS-containing DMEM), and then inoculated at a density of 5 x 10⁴ per well in a 48-well dish, and cultured for 48 hours. For adipocyte differentiation, 1 µg/mL human insulin (Sigma, Cat. No. 19278), 0.5 mM IBMX (3-isobutyl-1-methylxanthine, Sigma, Cat. No.15879), and 1 µM Dexamethasone (Sigma, Cat. No. D4902) were mixed with the differentiation medium, and 250 µl of the mixture was added to each well, after the previous medium was removed. After 48 hours, the medium was exchanged with the differentiation medium supplemented with only 1 µg/mL of human insulin. Thereafter, while the medium was exchanged with the differentiation medium supplemented with 1 µg/mL of human insulin every 48 hours, induction of adipocyte differentiation was examined for 7-9 days. To test glucose uptake, the differentiated cells were washed with serum-free DMEM medium once, and then 250 µl was added to induce serum depletion for 4 hours. Serum-free DMEM medium was used to carry out 10-fold serial dilutions for Human insulin from 2 µM to 0.01 µM, and for native insulin-immunoglobulin Fc conjugate and insulin analog-immunoglobulin Fc conjugates from 20 µM to 0.02 µM. Each 250 µl of the samples thus prepared were added to cells, and cultured in a 5% CO₂ incubator at 37°C for 24 hours. In order to measure the residual amount of glucose in the medium after incubation, 200 µl of the medium was taken and diluted 5-fold with D-PBS, followed by GOPOD (GOPOD Assay Kit, Megazyme, Cat. No. K-GLUC) assay. Based on the absorbance of glucose standard solution, the concentration of glucose remaining in the medium was converted, and EC50 values for glucose uptake of native insulin-immunoglobulin Fc conjugate and insulin analog-immunoglobulin Fc conjugates were calculated, respectively.

As a result, compared to human insulin, native insulin-immunoglobulin Fc conjugate showed glucose uptake of 11.6%, insulin analog (No. 6)-immunoglobulin Fc conjugate showed glucose uptake of 0.43%, insulin analog (No. 7)-immunoglobulin Fc conjugate showed glucose uptake of 1.84%, insulin analog (No. 8)-immunoglobulin Fc conjugate showed glucose uptake of 16.0%, insulin analog (No. 9)-immunoglobulin Fc conjugate showed glucose uptake of 15.1% (Table 5). As such, it was observed that the insulin analog (No. 6)-immunoglobulin Fc conjugate and insulin analog (No. 7)-immunoglobulin Fc conjugate of the present disclosure had remarkably reduced in vitro titer, compared to native insulin-immunoglobulin Fc conjugate, and insulin analog (No. 8)-immunoglobulin Fc conjugate and insulin analog (No. 9)-immunoglobulin Fc conjugate had in vitro titer similar to that of the native insulin-immunoglobulin Fc conjugate.

**[Table 5]**

| **Test No.** | **Substance name** | **Glucose uptake (relative to native insulin)** |
|---|---|---|
| Test 1 | Native human insulin | 100% |
| | **Native insulin-immunoglobulin Fc conjugate** | 11.6% |
| | **Insulin Analog No.6-immunoglobulin Fc conjugate** | 0.43% |
| | **Insulin Analog No.7-immunoglobulin Fc conjugate** | 1.84% |
| **Test 2** | **Native human insulin** | 100% |
| | **Native insulin-immunoglobulin Fc conjugate** | 15.2% |
| | **Insulin Analog No.8-immunoglobulin Fc conjugate** | 16.0% |
| **Test 3** | **Native human insulin** | 100% |
| | **Native insulin-immunoglobulin Fc conjugate** | 11.7% |
| | **Insulin Analog No.9-immunoglobulin Fc conjugate** | 15.1% |

### Example 11: Pharmacokinetics of insulin analog-immunoglobulin Fc conjugate

In order to examine pharmacokinetics of the insulin analog-immunoglobulin Fc conjugates, their blood concentration over time was compared in normal rats (SD rat, male, 6-week old) adapted for 5 days to the laboratory. 21.7 nmol/kg of native insulin-immunoglobulin Fc conjugate and 65.1 nmol/kg of insulin analog-immunoglobulin Fc conjugate were subcutaneously injected, respectively. The blood was collected at 0, 1, 4, 8, 24, 48, 72, 96, 120, 144, 168, 192, and 216 hours. At each time point, blood concentrations of native insulin-immunoglobulin Fc conjugate and insulin analog-immunoglobulin Fc conjugate were measured by enzyme linked immunosorbent assay (ELISA), and Insulin ELISA (ALPCO, USA) was used as a kit. However, as a detection antibody, mouse anti-human IgG4 HRP conjugate (Alpha Diagnostic Intl, Inc, USA) was used.

The results of examining pharmacokinetics of the native insulin-immunoglobulin Fc conjugate and the insulin analog-immunoglobulin Fc conjugate showed that their blood concentrations increased in proportion to their administration concentrations, and the insulin analog-immunoglobulin Fc conjugates having low insulin receptor binding affinity showed highly increased half-life, compared to the native insulin-Fc conjugate (FIG. 6).

These results suggest that when the insulin analogs of the present disclosure modified to have reduced insulin receptor binding affinity are linked to immunoglobulin Fc region to prepare conjugates, the conjugates can be provided as stable insulin formulations due to remarkably increased in vivo blood half-life, and thus effectively used as therapeutic agents for diabetes. Furthermore, since the insulin analogs according to the present disclosure themselves also have reduced insulin receptor binding affinity and reduced titer, the insulin analogs also exhibit the same effect although they are linked to other various carriers.
## Claims

1. An insulin analog conjugate, in which (i) an insulin analog having a reduced insulin titer compared to the native form, wherein an amino acid in B chain of insulin is modified by substituting one amino acid selected from the group consisting of 24th amino acid and 25th amino acid of B chain with alanine;
is linked to (ii) a biocompatible material, wherein the biocompatible material is an immunoglobulin Fc region.

2. The insulin analog conjugate according to claim 1, wherein the insulin analog is selected from the group consisting of SEQ ID NOs. 30, and 32.

3. The insulin analog conjugate according to claim 1, wherein the insulin analog and the biocompatible material are linked to each other via a peptide or a non-peptidyl polymer as a linker.

4. The insulin analog conjugate according to claim 1, wherein (i) the insulin analog is linked to (ii) the immunoglobulin Fc region via (iii) a peptide linker or a non-peptidyl linker selected from the group consisting of polyethylene glycol, polypropylene glycol, copolymers of ethylene glycol-propylene glycol, polyoxyethylated polyols, polyvinyl alcohol, polysaccharides, dextran, polyvinyl ethyl ether, biodegradable polymers, lipid polymers, chitins, hyaluronic acid and combination thereof.

5. The insulin analog conjugate according to claim 4, wherein the non-peptidyl linker is linked to the N-terminus of B chain of the insulin analog.

6. The insulin analog conjugate according to claim 4, wherein both ends of the non-peptidyl polymer are linked to the N-terminus of the immunoglobulin Fc region and the N-terminal amine group of the insulin analog or the ε-amino group of the internal lysine residue or thiol group of B chain, respectively.

7. The insulin analog conjugate according to claim 4, wherein the immunoglobulin Fc region is aglycosylated,
optionally wherein:
(i) the immunoglobulin Fc region is composed of 1 domain to 4 domains selected from the group consisting of CH1, CH2, CH3 and CH4 domains; or
(iii) the immunoglobulin Fc region further includes a hinge region.

8. The insulin analog according to claim 4, wherein the immunoglobulin Fc region is an Fc region derived from IgG, IgA, IgD, IgE or IgM.

9. The insulin analog conjugate according to claim 8, wherein:
(i) each domain of the immunoglobulin Fc region is a hybrid of domains having different origins and being derived from an immunoglobulin selected from the group consisting of IgG, IgA, IgD, IgE and IgM;
(ii) the immunoglobulin Fc region is a dimer or a multimer consisting of single-chain immunoglobulins composed of domains of the same origin; or
(iii) the immunoglobulin Fc region is an IgG4 Fc region, optionally wherein the immunoglobulin Fc region is a human IgG4-derived aglycosylated Fc region.

10. The insulin analog conjugate according to claim 4, wherein the reactive group of the non-peptidyl linker is selected from the group consisting of an aldehyde group, a propionaldehyde group, a butyraldehyde group, a maleimide group and a succinimide derivative,
optionally wherein:
(i) the reactive group of the non-peptidyl linker is succinimidyl propionate, succinimidyl carboxymethyl, hydroxy succinimidyl, or succinimidyl carbonate; or
(ii) the non-peptidyl linker has reactive aldehyde groups at both ends thereof.

11. A long-acting insulin formulation having improved in vivo duration and stability, comprising the insulin analog conjugate according to any one of claims 1 to 10.

12. The long-acting insulin formulation according to claim 11, wherein the formulation is a therapeutic agent for diabetes.

13. A method for preparing the insulin analog conjugate according to any one of claims 1 to 10, comprising:
linking an insulin analog having a reduced insulin titer compared to the native form, wherein an amino acid in B chain of insulin is modified by substituting one amino acid selected from the group consisting of 24th amino acid and 25th amino acid of B chain with alanine;
to a biocompatible material, wherein the biocompatible material is an immunoglobulin Fc region.

## Patentansprüche

1. Insulinanalogonkonjugat, bei dem (i) ein Insulinanalogon mit einem verringerten Insulintiter im Vergleich zur nativen Form, wobei eine Aminosäure in der B-Kette von Insulin durch Substitution einer Aminosäure, die aus der aus der 24. Aminosäure und der 25. Aminosäure der B-Kette ausgewählt ist, mit Alanin modifiziert ist;
an (ii) ein biokompatibles Material gebunden ist, wobei das biokompatible Material eine Immunglobulin-Fc-Region ist.

2. Insulinanalogonkonjugat nach Anspruch 1, wobei das Insulinanalogon aus der aus SEQ ID NO: 30 und 32 bestehenden Gruppe ausgewählt ist.

3. Insulinanalogonkonjugat nach Anspruch 1, wobei das Insulinanalogon und das biokompatible Material über ein Peptid oder ein Nichtpeptidylpolymer als Linker miteinander verbunden sind.

4. Insulinanalogonkonjugat nach Anspruch 1, wobei (i) das Insulinanalogon an (ii) die Immunglobulin-Fc-Region über (iii) einen Peptidlinker oder einen Nichtpeptidyllinker gebunden ist, der aus der aus Polyethylenglykol, Polypropylenglykol, Copolymeren von Ethylenglykol-Propylenglykol, polyoxyethylierten Polyolen, Polyvinylalkohol, Polysacchariden, Dextran, Polyvinylethylether, biologisch abbaubaren Polymeren, Lipidpolymeren, Chitinen, Hyaluronsäure und Kombinationen davon bestehenden Gruppe ausgewählt ist.

5. Insulinanalogonkonjugat nach Anspruch 4, wobei der Nichtpeptidyllinker an den N-Terminus der B-Kette des Insulinanalogons gebunden ist.

6. Insulinanalogonkonjugat nach Anspruch 4, wobei beide Enden des Nichtpeptidylpolymers an den N-Terminus der Immunglobulin-Fc-Region und die N-terminale Amingruppe des Insulinanalogons oder die ε-Aminogruppe des internen Lysinrests bzw. die Thiolgruppe der B-Kette gebunden sind.

7. Insulinanalogonkonjugat nach Anspruch 4, wobei die Immunglobulin-Fc-Region aglykosyliert ist,
wobei gegebenenfalls
(i) die Immunglobulin-Fc-Region aus 1 Domäne bis 4 Domänen besteht, die aus der aus der CH1-, CH2-, CH3- und CH4-Domäne bestehenden Gruppe ausgewählt sind; oder
(iii) die Immunglobulin-Fc-Region weiters eine Gelenkregion umfasst.

8. Insulinanalogon nach Anspruch 4, wobei die Immunglobulin-Fc-Region eine von IgG, IgA, IgD, IgE oder IgM abgeleitete Fc-Region ist.

9. Insulinanalogonkonjugat nach Anspruch 8, wobei:
(i) jede Domäne der Immunglobulin-Fc-Region ein Hybrid von Domänen mit unterschiedlichem Ursprung ist und von einem Immunglobulin abgeleitet ist, das aus der aus IgG, IgA, IgD, IgE und IgM bestehenden Gruppe ausgewählt ist;
(ii) die Immunglobulin-Fc-Region ein Dimer oder ein Multimer ist, das aus einkettigen Immunglobulinen besteht, die aus Domänen desselben Ursprungs bestehen; oder
(iii) die Immunglobulin-Fc-Region eine IgG4-Fc-Region ist, wobei gegebenenfalls die Immunglobulin-Fc-Region eine von menschlichem IgG4 abgeleitete, aglykosylierte Fc-Region ist.

10. Insulinanalogonkonjugat nach Anspruch 4, wobei die reaktive Gruppe des Nichtpeptidyllinkers aus der aus einer Aldehydgruppe, einer Propionaldehydgruppe, einer Butyraldehydgruppe, einer Maleinimidgruppe und einem Succinimidderivat bestehenden Gruppe ausgewählt ist, wobei gegebenenfalls:
(i) die reaktive Gruppe des Nichtpeptidyllinkers Succinimidylpropionat, Succinimidylcarboxymethyl, Hydroxysuccinimidyl oder Succinimidylcarbonat ist; oder
(ii) der Nichtpeptidyllinker reaktive Aldehydgruppen an seinen beiden Enden aufweist.

11. Lang wirkende Insulinformulierung mit verbesserter In-vivo-Dauer und -Stabilität, die ein Insulinanalogonkonjugat nach einem der Ansprüche 1 bis 10 umfasst.

12. Lang wirkende Insulinformulierung nach Anspruch 11, wobei die Formulierung ein Therapeutikum für Diabetes ist.

13. Verfahren zur Herstellung eines Insulinanalogonkonjugats nach einem der Ansprüche 1 bis 10, das Folgendes umfasst:
Binden eines Insulinanalogons mit einem verringerten Insulintiter im Vergleich zur nativen Form, wobei eine Aminosäure in der B-Kette von Insulin durch Substitution einer Aminosäure, die aus der aus der 24. Aminosäure und der 25. Aminosäure der B-Kette ausgewählt ist, mit Alanin modifiziert ist;
an ein biokompatibles Material, wobei das biokompatible Material eine Immunglobulin-Fc-Region ist.

## Revendications

1. Conjugué d'analogue d'insuline, dans lequel (i) un analogue d'insuline ayant un titre d'insuline réduit par rapport à la forme native, dans lequel un acide aminé dans la chaîne B de l'insuline est modifié par substitution d'un acide aminé choisi dans le groupe constitué du 24^{e} acide aminé et du 25^{e} acide aminé de la chaîne B par l'alanine ;
est lié à (ii) un matériau biocompatible, dans lequel le matériau biocompatible est une région Fc d'immunoglobuline.

2. Conjugué d'analogue d'insuline selon la revendication 1, dans lequel l'analogue d'insuline est choisi dans le groupe constitué de SEQ ID NO. 30 et 32.

3. Conjugué d'analogue d'insuline selon la revendication 1, dans lequel l'analogue d'insuline et le matériau biocompatible sont liés l'un à l'autre par l'intermédiaire d'un peptide ou d'un polymère non-peptidyle en tant que lieur.

4. Conjugué d'analogue d'insuline selon la revendication 1, dans lequel (i) l'analogue d'insuline est lié à (ii) la région Fc d'immunoglobuline par l'intermédiaire de (iii) un lieur peptidique ou un lieur non-peptidyle choisi dans le groupe constitué des polyéthylène glycol, polypropylène glycol, copolymères d'éthylène glycol-propylène glycol, polyols polyoxyéthylés, alcool polyvinylique, polysaccharides, dextrane, éther éthylique de polyvinyle, polymères biodégradables, polymères lipidiques, chitines, acide hyaluronique et une combinaison de ceux-ci.

5. Conjugué d'analogue d'insuline selon la revendication 4, dans lequel le lieur non-peptidyle est lié à l'extrémité N-terminale de la chaîne B de l'analogue d'insuline.

6. Conjugué d'analogue d'insuline selon la revendication 4, dans lequel les deux extrémités du polymère non-peptidyle sont liées à l'extrémité N-terminale de la région Fc d'immunoglobuline et au groupe amine N-terminal de l'analogue d'insuline ou au groupe ε-amino du résidu lysine interne ou au groupe thiol de la chaîne B, respectivement.

7. Conjugué d'analogue d'insuline selon la revendication 4, dans lequel la région Fc d'immunoglobuline est aglycosylée,
facultativement dans lequel :
(i) la région Fc d'immunoglobuline est composée de 1 domaine à 4 domaines choisis dans le groupe constitué de domaines CH1, CH2, CH3 et CH4 ; ou
(iii) la région Fc d'immunoglobuline comprend en outre une région de charnière.

8. Analogue d'insuline selon la revendication 4, dans lequel la région Fc d'immunoglobuline est une région Fc dérivée de IgG, IgA, IgD, IgE ou IgM.

9. Conjugué d'analogue d'insuline selon la revendication 8, dans lequel :
(i) chaque domaine de la région Fc d'immunoglobuline est un hybride de domaines ayant différentes origines et étant dérivés d'une immunoglobuline choisie dans le groupe constitué de IgG, IgA, IgD, IgE et IgM ;
(ii) la région Fc d'immunoglobuline est un dimère ou un multimère constitué d'immunoglobulines monocaténaires composées de domaines de la même origine ; ou
(iii) la région Fc d'immunoglobuline est une région Fc d'IgG4, facultativement dans lequel la région Fc d'immunoglobuline est une région Fc aglycosylée dérivée d'IgG4 humaine.

10. Conjugué d'analogue d'insuline selon la revendication 4, dans lequel le groupe réactif du lieur non-peptidyle est choisi dans le groupe constitué d'un groupe aldéhyde, un groupe propionaldéhyde, un groupe butyraldéhyde, un groupe maléimide et un dérivé de succinimide,
facultativement dans lequel :
(i) le groupe réactif du lieur non-peptidyle est propionate de succinimidyle, succinimidylcarboxyméthyle, hydroxysuccinimidyle ou carbonate de succinimidyle ; ou
(ii) le lieur non-peptidyle comporte des groupes aldéhyde réactifs aux deux extrémités de celui-ci.

11. Formulation d'insuline à action prolongée ayant une durée d'action et une stabilité *in vivo*, comprenant le conjugué d'analogue d'insuline selon l'une quelconque des revendications 1 à 10.

12. Formulation d'insuline à action prolongée selon la revendication 11, où la formulation est un agent thérapeutique pour le diabète.

13. Procédé de préparation du conjugué d'analogue d'insuline selon l'une quelconque des revendications 1 à 10, comprenant :
la liaison d'un analogue d'insuline ayant un titre d'insuline réduit par rapport à la forme native, dans lequel un acide aminé dans la chaîne B de l'insuline est modifié par substitution d'un acide aminé choisi dans le groupe constitué du 24^{e} acide aminé et du 25^{e} acide aminé de la chaîne B par l'alanine ;
à un matériau biocompatible, dans lequel le matériau biocompatible est une région Fc d'immunoglobuline.
